(19) Europäisches Patentamt
European Patent Office
Office européen des brevets

(11) EP 1 319 715 B1

(12) EUROPÄISCHE PATENTSCHRIFT

(45) Veröffentlichungstag und Bekanntmachung des Hinweises auf die Patenterteilung:
**17.08.2005 Patentblatt 2005/33**

(51) Int Cl.[7]: **C12Q 1/32**, C12N 9/04, C12N 15/53, C12N 1/15, C12N 1/21, C12N 5/10, C07K 16/14

(21) Anmeldenummer: **02026487.5**

(22) Anmeldetag: **28.11.2002**

(54) **IMP Dehydrogenase und deren Verwendung zum Identifizieren von fungizid wirksamen Verbindungen**

IMP dehydrogenase and its use for the identification of fungicidal substances

IMP déshydrogénase et son utilisation pour l'identification de substances fongicides

(84) Benannte Vertragsstaaten:
**AT BE BG CH CY CZ DE DK EE ES FI FR GB GR IE IT LI LU MC NL PT SE SK TR**

(30) Priorität: **11.12.2001 DE 10160660**

(43) Veröffentlichungstag der Anmeldung:
**18.06.2003 Patentblatt 2003/25**

(73) Patentinhaber: **Bayer CropScience AG**
**40789 Monheim (DE)**

(72) Erfinder:
- **Aichinger, Christian, Dr.**
  **50939 Köln (DE)**
- **Schreier, Peter, Prof.Dr.**
  **50674 Köln (DE)**
- **Li, Volkhart, Dr.**
  **42553 Velbert (DE)**
- **Voerste, Arnd, Dr.**
  **50677 Köln (DE)**
- **Kuck, Karl-Heinz, Dr.**
  **40764 Langenfeld (DE)**

(56) Entgegenhaltungen:
**WO-A-99/33996**

- **KÖHLER G A ET AL: "Overexpression of a cloned IMP dehydrogenase gene of Candida albicans confers resistance to the specific inhibitor mycophenolic acid." JOURNAL OF BACTERIOLOGY. UNITED STATES APR 1997, Bd. 179, Nr. 7, April 1997 (1997-04), Seiten 2331-2338, XP002234556 ISSN: 0021-9193**
- **O'GARA M J ET AL: "IMP dehydrogenase from Pneumocystis carinii as a potential drug target." ANTIMICROBIAL AGENTS AND CHEMOTHERAPY. UNITED STATES JAN 1997, Bd. 41, Nr. 1, Januar 1997 (1997-01), Seiten 40-48, XP002234557 ISSN: 0066-4804**
- **FARAZI T ET AL: "Isolation and characterization of mycophenolic acid-resistant mutants of inosine-5'-monophosphate dehydrogenase." THE JOURNAL OF BIOLOGICAL CHEMISTRY. UNITED STATES 10 JAN 1997, Bd. 272, Nr. 2, 10. Januar 1997 (1997-01-10), Seiten 961-965, XP002234558 ISSN: 0021-9258**
- **ANDERSON W K ET AL: "Synthesis and modeling studies with monocyclic analogues of mycophenolic acid." JOURNAL OF MEDICINAL CHEMISTRY. UNITED STATES 5 JAN 1996, Bd. 39, Nr. 1, 5. Januar 1996 (1996-01-05), Seiten 46-55, XP002234559 ISSN: 0022-2623**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

SEQUENCE LISTING

<110> Bayer AG

<120> Polypeptide zum Identifizieren von fungizid wirksamen Verbindungen

<130> Le A 35 733

<160> 6

<170> PatentIn version 3.1

<210> 1
<211> 3063

<212> DNA
<213> Ustilago maydis

<220>
<221> CDS
<222> (582)..(1071)
<223>

<220>
<221> CDS
<222> (1412)..(2580)
<223>

<400> 1

```
ttcacgattt acgatttacg attcgcgatt cacgattcat gattcacgat tcacgacgtt      60

ttacgatgtg atttgtggtg caagcgcgca ctctctcgac gattgaacgg agcaaactcg     120

tcgggcgtaa ctcgtgactg tagctcgtgg ctagacgctt gccaggtgag acgggctcgg     180

tttgcttgac gtgttcacat cgcgtgggtt gcggcgctag gtcaatcacg aatccttttt     240

cattcaatca ctgaatgata atataatttt gcccacctta gagaaagaag aaacgaaagc     300

cttaatcccg atctggcagc tagatttttt gatttttttt tttcgcttac acaaactgtg     360

agagggagag agttgaaact tttttttcc ctacatggtg gctaaaagtg ggctagggtt     420

actctccctc tcgtggtcgt tagcagcgaa gagaagcgaa gcgaagtaca aaggcatcca     480

atgccagagc gacatcttcc ttcttaaact gttcttcgcc aaaagagcca cccacttcca     540

acataccttt cctcgcccctt cacctcccat catccatcaa a atg cct gct agc aac     596
                                               Met Pro Ala Ser Asn
                                               1               5
```

ggt att cag ctc cct cag gac gaa gcg gtc ctt tcg cct tct cag gcg 644
Gly Ile Gln Leu Pro Gln Asp Glu Ala Val Leu Ser Pro Ser Gln Ala
10 15 20

ctt gag cac ctc aag acc tac act tac ggc gat ggt ctc agc atg gcc 692
Leu Glu His Leu Lys Thr Tyr Thr Tyr Gly Asp Gly Leu Ser Met Ala
25 30 35

gag ctc atc gac tcg cgt cag cac ggt ggt ctc acc tac aat gac ttt 740
Glu Leu Ile Asp Ser Arg Gln His Gly Gly Leu Thr Tyr Asn Asp Phe
40 45 50

ctc gtt ctg ccc ggt ttc atc aac ttt gct gct tcg gac gtc agc ctg 788
Leu Val Leu Pro Gly Phe Ile Asn Phe Ala Ala Ser Asp Val Ser Leu
55 60 65

cgc acc aag gtg acc aag aac gtt acg ctc aac acg cct ttc ctc tcg 836
Arg Thr Lys Val Thr Lys Asn Val Thr Leu Asn Thr Pro Phe Leu Ser
70 75 80 85

tcg ccc atg gac acg gtt acc gag acc gag atg gcg atc gca atg ggc 884
Ser Pro Met Asp Thr Val Thr Glu Thr Glu Met Ala Ile Ala Met Gly
90 95 100

ttg atg ggc ggt atg ggt gtc att cac aac aac atg agc cct cag gag 932
Leu Met Gly Gly Met Gly Val Ile His Asn Asn Met Ser Pro Gln Glu
105 110 115

cag gct agc gtt gtg cgc aag gtc aag aag tac gag aac ggt ttc atc 980
Gln Ala Ser Val Val Arg Lys Val Lys Lys Tyr Glu Asn Gly Phe Ile
120 125 130

acc gaa cct ctc tgc ctc gac ccc aag gcc acc gtc ggt gac gtt ctc 1028
Thr Glu Pro Leu Cys Leu Asp Pro Lys Ala Thr Val Gly Asp Val Leu
135 140 145

```
gat gtc aag gag cgt ctg ggt ttt ggt ggt att cct atc act g          1071
Asp Val Lys Glu Arg Leu Gly Phe Gly Gly Ile Pro Ile Thr
150                 155                 160

gtaagttgtc gatcgaatta aaacagcttt ctacttcttc tttctcctcc tcctcccctt  1131

ccccggacta cccgtcccgc tgcaccgctc gacaatcttg gtcgacctcg cctcttcact  1191

gctacgctct cactcaccaa aggatgaaac aaccgtattg catataacct tcgttcttcc  1251

gagcctctcc cttagacttg ggagagtgtg tgcggatgcg atttttcaac tctgatttgc  1311

ctctcattcg atcgctgttc ggtctcgaca tggttctctc gatcttttat gctgacctcg  1371

atcttctctg tgtttcgcat accccatcga tccgacgtag ac  act ggt gcg atg     1425
                                            Asp Thr Gly Ala Met
                                                165

cac ggc aag ctt ctc ggt atc gtc act gct cgt gac gtc cag ttc cgt    1473
His Gly Lys Leu Leu Gly Ile Val Thr Ala Arg Asp Val Gln Phe Arg
    170                 175                 180

gat acc acg ctt ccg ctt tcc gag gtc atg acc acc gac ctt gtc acc    1521
Asp Thr Thr Leu Pro Leu Ser Glu Val Met Thr Thr Asp Leu Val Thr
185                 190                 195                 200

gcc aag cag gga gtc acg ctc gag cag gcc aac act atc ctg cgt gac    1569
Ala Lys Gln Gly Val Thr Leu Glu Gln Ala Asn Thr Ile Leu Arg Asp
                205                 210                 215

agc aaa aag ggc aag ctc ccc atc gtc gac gcc gag ggc cgc ctt gtt    1617
Ser Lys Lys Gly Lys Leu Pro Ile Val Asp Ala Glu Gly Arg Leu Val
            220                 225                 230

gcc ctg ctc gct cgc tct gac ttg ctc aag aat caa aac ttc cct ctc    1665
```

Ala Leu Leu Ala Arg Ser Asp Leu Leu Lys Asn Gln Asn Phe Pro Leu
235 240 245

gcc tcc aag cgt ccc gaa agc aag cag ctt tac tgt gcc gct gcc atc 1713
Ala Ser Lys Arg Pro Glu Ser Lys Gln Leu Tyr Cys Ala Ala Ala Ile
250 255 260

ggc act cgt ccc tca gac cgt gaa cgt ctc agt ctt ctt gta gag gct 1761
Gly Thr Arg Pro Ser Asp Arg Glu Arg Leu Ser Leu Leu Val Glu Ala
265 270 275 280

gga ttg gac gtt gtc atc ctc gac tcg tcc cag ggt aac tcg gtg tat 1809
Gly Leu Asp Val Val Ile Leu Asp Ser Ser Gln Gly Asn Ser Val Tyr
285 290 295

cag atc gaa atg atc cag tgg atc aag cag acc tac ccg cag atc gac 1857
Gln Ile Glu Met Ile Gln Trp Ile Lys Gln Thr Tyr Pro Gln Ile Asp
300 305 310

gtt gtc gcc ggt aac gtc gtc aca cga gag cag gct gcc agc ctg atc 1905
Val Val Ala Gly Asn Val Val Thr Arg Glu Gln Ala Ala Ser Leu Ile
315 320 325

gcc gct ggt gct gac gcc ctt cgt gtc ggc atg ggt tcc ggt tcg atc 1953
Ala Ala Gly Ala Asp Ala Leu Arg Val Gly Met Gly Ser Gly Ser Ile
330 335 340

tgc atc acc cag gaa gtg atg gct gtc ggt cga cct cag ggt acc gcc 2001
Cys Ile Thr Gln Glu Val Met Ala Val Gly Arg Pro Gln Gly Thr Ala
345 350 355 360

gtc cac gcc gtt gct gag ttc gcc tcc aag ttt ggc gtc ccc gtc atc 2049
Val His Ala Val Ala Glu Phe Ala Ser Lys Phe Gly Val Pro Val Ile
365 370 375

gcc gat ggt gga att tcc aat gtc ggt cac atc gcc aaa gct ctc gca 2097

Ala Asp Gly Gly Ile Ser Asn Val Gly His Ile Ala Lys Ala Leu Ala
380 385 390

ctc ggt gct tcc gcc gtc atg atg gga ggc ttg ctc gcc gga acc aac 2145
Leu Gly Ala Ser Ala Val Met Met Gly Gly Leu Leu Ala Gly Thr Asn
395 400 405

gaa tcc ccc ggt gac tac ttc tat cgc gac ggt aag cgt ctc aag ggt 2193
Glu Ser Pro Gly Asp Tyr Phe Tyr Arg Asp Gly Lys Arg Leu Lys Gly
410 415 420

tac cgt ggt atg gga tcc atc gaa gcc atg gag cac cag aag aag ggc 2241
Tyr Arg Gly Met Gly Ser Ile Glu Ala Met Glu His Gln Lys Lys Gly
425 430 435 440

aag atc gcc ggc gcc acc ggt aaa ggt gct gcc aag gct gac aag gtt 2289
Lys Ile Ala Gly Ala Thr Gly Lys Gly Ala Ala Lys Ala Asp Lys Val
445 450 455

gct acc gac gaa aac gcc gct acg cag cga tac ttt tct gaa agc gac 2337
Ala Thr Asp Glu Asn Ala Ala Thr Gln Arg Tyr Phe Ser Glu Ser Asp
460 465 470

gcc gtc aag gtc gcc cag ggc gtt gca ggt gct gtg cag gac aag ggc 2385
Ala Val Lys Val Ala Gln Gly Val Ala Gly Ala Val Gln Asp Lys Gly
475 480 485

tcg gtc aag aag ttc ttg cct tac ctg tac acc ggt ctg caa cac tcg 2433
Ser Val Lys Lys Phe Leu Pro Tyr Leu Tyr Thr Gly Leu Gln His Ser
490 495 500

ttg cag gac atg ggt gtc cca cac ctc tac cag ttg cgc tct gca gtg 2481
Leu Gln Asp Met Gly Val Pro His Leu Tyr Gln Leu Arg Ser Ala Val
505 510 515 520

gcc tcg ggc cag gtg agg ttc gag ttg agg acc gca agc gcc cag gtc 2529

```
Ala Ser Gly Gln Val Arg Phe Glu Leu Arg Thr Ala Ser Ala Gln Val
                525                 530                 535

gag ggt ggt gtc cac ggg ctt cac agc tac gag aag cgt ctg ttc tct      2577
Glu Gly Gly Val His Gly Leu His Ser Tyr Glu Lys Arg Leu Phe Ser
            540                 545                 550

tcg tagggttcga gttgaggacc gcaagcgccc aggtcgaggg tggtgtccac           2630
Ser

gggcttcaca gctacgagaa gcgtctgttc tcttcgtaga tgtttccctt ttaagaagca    2690

cccatctttg gcaagacaaa caatctcatt ttgtctcttg ccaaatcgaa accatgaggc    2750

tcgaccggac ccatcgtgtc aacaaagatc tcagctttgt ggccttgtct acacgtgcca    2810

gcagctcgcc tctctatctc tatcatttgt actttagtcg cttttgtttc acccttcatt    2870

ctgctcaaaa gatgtgttat gaatcgtgat tctagcctag cgctcttttc agcgctgctt    2930

ggcaaagtct gtggatgcag ttgaagtgag tcacagagta aaagcgagtg gatctttggt    2990

gcctgtttgg atgagttgaa gtaagatcga gaaatgttag ggtacgcgaa ctttcgagaa    3050

agacttcggg gaa                                                       3063
```

<210> 2
<211> 553
<212> PRT
<213> Ustilago maydis

<400> 2

Met Pro Ala Ser Asn Gly Ile Gln Leu Pro Gln Asp Glu Ala Val Leu
1 5 10 15

Ser Pro Ser Gln Ala Leu Glu His Leu Lys Thr Tyr Thr Tyr Gly Asp
20 25 30

Gly Leu Ser Met Ala Glu Leu Ile Asp Ser Arg Gln His Gly Gly Leu
35 40 45

Thr Tyr Asn Asp Phe Leu Val Leu Pro Gly Phe Ile Asn Phe Ala Ala
50 55 60

Ser Asp Val Ser Leu Arg Thr Lys Val Thr Lys Asn Val Thr Leu Asn
65 70 75 80

Thr Pro Phe Leu Ser Ser Pro Met Asp Thr Val Thr Glu Thr Glu Met
85 90 95

Ala Ile Ala Met Gly Leu Met Gly Gly Met Gly Val Ile His Asn Asn
100 105 110

Met Ser Pro Gln Glu Gln Ala Ser Val Val Arg Lys Val Lys Lys Tyr
115 120 125

Glu Asn Gly Phe Ile Thr Glu Pro Leu Cys Leu Asp Pro Lys Ala Thr
130 135 140

Val Gly Asp Val Leu Asp Val Lys Glu Arg Leu Gly Phe Gly Gly Ile
145 150 155 160

Pro Ile Thr Asp Thr Gly Ala Met His Gly Lys Leu Leu Gly Ile Val
165 170 175

Thr Ala Arg Asp Val Gln Phe Arg Asp Thr Thr Leu Pro Leu Ser Glu
180 185 190

Val Met Thr Thr Asp Leu Val Thr Ala Lys Gln Gly Val Thr Leu Glu
195 200 205

Gln Ala Asn Thr Ile Leu Arg Asp Ser Lys Lys Gly Lys Leu Pro Ile
210 215 220

Val Asp Ala Glu Gly Arg Leu Val Ala Leu Leu Ala Arg Ser Asp Leu
225 230 235 240

Leu Lys Asn Gln Asn Phe Pro Leu Ala Ser Lys Arg Pro Glu Ser Lys
245 250 255

Gln Leu Tyr Cys Ala Ala Ala Ile Gly Thr Arg Pro Ser Asp Arg Glu
260 265 270

Arg Leu Ser Leu Leu Val Glu Ala Gly Leu Asp Val Val Ile Leu Asp
275 280 285

Ser Ser Gln Gly Asn Ser Val Tyr Gln Ile Glu Met Ile Gln Trp Ile
290 295 300

Lys Gln Thr Tyr Pro Gln Ile Asp Val Val Ala Gly Asn Val Val Thr
305 310 315 320

Arg Glu Gln Ala Ala Ser Leu Ile Ala Ala Gly Ala Asp Ala Leu Arg
325 330 335

Val Gly Met Gly Ser Gly Ser Ile Cys Ile Thr Gln Glu Val Met Ala
340 345 350

Val Gly Arg Pro Gln Gly Thr Ala Val His Ala Val Ala Glu Phe Ala
355 360 365

Ser Lys Phe Gly Val Pro Val Ile Ala Asp Gly Gly Ile Ser Asn Val
370 375 380

Gly His Ile Ala Lys Ala Leu Ala Leu Gly Ala Ser Ala Val Met Met
385 390 395 400

Gly Gly Leu Leu Ala Gly Thr Asn Glu Ser Pro Gly Asp Tyr Phe Tyr
405 410 415

Arg Asp Gly Lys Arg Leu Lys Gly Tyr Arg Gly Met Gly Ser Ile Glu
420 425 430

Ala Met Glu His Gln Lys Lys Gly Lys Ile Ala Gly Ala Thr Gly Lys
435 440 445

Gly Ala Ala Lys Ala Asp Lys Val Ala Thr Asp Glu Asn Ala Ala Thr
450 455 460

Gln Arg Tyr Phe Ser Glu Ser Asp Ala Val Lys Val Ala Gln Gly Val
465 470 475 480

Ala Gly Ala Val Gln Asp Lys Gly Ser Val Lys Lys Phe Leu Pro Tyr
485 490 495

Leu Tyr Thr Gly Leu Gln His Ser Leu Gln Asp Met Gly Val Pro His
500 505 510

Leu Tyr Gln Leu Arg Ser Ala Val Ala Ser Gly Gln Val Arg Phe Glu
515 520 525

Leu Arg Thr Ala Ser Ala Gln Val Glu Gly Gly Val His Gly Leu His
530 535 540

Ser Tyr Glu Lys Arg Leu Phe Ser Ser
545 550

<210> 3
<211> 1662
<212> DNA

<213> Ustilago maydis

<220>
<221> CDS
<222> (1)..(1659)

<400> 3

```
atg cct gct agc aac ggt att cag ctc cct cag gac gaa gcg gtc ctt        48
Met Pro Ala Ser Asn Gly Ile Gln Leu Pro Gln Asp Glu Ala Val Leu
1               5                   10                  15

tcg cct tct cag gcg ctt gag cac ctc aag acc tac act tac ggc gat        96
Ser Pro Ser Gln Ala Leu Glu His Leu Lys Thr Tyr Thr Tyr Gly Asp
            20                  25                  30

ggt ctc agc atg gcc gag ctc atc gac tcg cgt cag cac ggt ggt ctc       144
Gly Leu Ser Met Ala Glu Leu Ile Asp Ser Arg Gln His Gly Gly Leu
        35                  40                  45

acc tac aat gac ttt ctc gtt ctg ccc ggt ttc atc aac ttt gct gct       192
Thr Tyr Asn Asp Phe Leu Val Leu Pro Gly Phe Ile Asn Phe Ala Ala
    50                  55                  60
```

```
tcg gac gtc agc ctg cgc acc aag gtg acc aag aac gtt acg ctc aac      240
Ser Asp Val Ser Leu Arg Thr Lys Val Thr Lys Asn Val Thr Leu Asn
65                  70                  75                  80

acg cct ttc ctc tcg tcg ccc atg gac acg gtt acc gag acc gag atg      288
Thr Pro Phe Leu Ser Ser Pro Met Asp Thr Val Thr Glu Thr Glu Met
                85                  90                  95

gcg atc gca atg ggc ttg atg ggc ggt atg ggt gtc att cac aac aac      336
Ala Ile Ala Met Gly Leu Met Gly Gly Met Gly Val Ile His Asn Asn
            100                 105                 110

atg agc cct cag gag cag gct agc gtt gtg cgc aag gtc aag aag tac      384
Met Ser Pro Gln Glu Gln Ala Ser Val Val Arg Lys Val Lys Lys Tyr
        115                 120                 125

gag aac ggt ttc atc acc gaa cct ctc tgc ctc gac ccc aag gcc acc      432
Glu Asn Gly Phe Ile Thr Glu Pro Leu Cys Leu Asp Pro Lys Ala Thr
    130                 135                 140

gtc ggt gac gtt ctc gat gtc aag gag cgt ctg ggt ttt ggt ggt att      480
Val Gly Asp Val Leu Asp Val Lys Glu Arg Leu Gly Phe Gly Gly Ile
145                 150                 155                 160

cct atc act gac act ggt gcg atg cac ggc aag ctt ctc ggt atc gtc      528
Pro Ile Thr Asp Thr Gly Ala Met His Gly Lys Leu Leu Gly Ile Val
                165                 170                 175

act gct cgt gac gtc cag ttc cgt gat acc acg ctt ccg ctt tcc gag      576
Thr Ala Arg Asp Val Gln Phe Arg Asp Thr Thr Leu Pro Leu Ser Glu
            180                 185                 190

gtc atg acc acc gac ctt gtc acc gcc aag cag gga gtc acg ctc gag      624
Val Met Thr Thr Asp Leu Val Thr Ala Lys Gln Gly Val Thr Leu Glu
        195                 200                 205
```

```
cag gcc aac act atc ctg cgt gac agc aaa aag ggc aag ctc ccc atc      672
Gln Ala Asn Thr Ile Leu Arg Asp Ser Lys Lys Gly Lys Leu Pro Ile
    210                 215                 220

gtc gac gcc gag ggc cgc ctt gtt gcc ctg ctc gct cgc tct gac ttg      720
Val Asp Ala Glu Gly Arg Leu Val Ala Leu Leu Ala Arg Ser Asp Leu
225                 230                 235                 240

ctc aag aat caa aac ttc cct ctc gcc tcc aag cgt ccc gaa agc aag      768
Leu Lys Asn Gln Asn Phe Pro Leu Ala Ser Lys Arg Pro Glu Ser Lys
                245                 250                 255

cag ctt tac tgt gcc gct gcc atc ggc act cgt ccc tca gac cgt gaa      816
Gln Leu Tyr Cys Ala Ala Ala Ile Gly Thr Arg Pro Ser Asp Arg Glu
            260                 265                 270

cgt ctc agt ctt ctt gta gag gct gga ttg gac gtt gtc atc ctc gac      864
Arg Leu Ser Leu Leu Val Glu Ala Gly Leu Asp Val Val Ile Leu Asp
        275                 280                 285

tcg tcc cag ggt aac tcg gtg tat cag atc gaa atg atc cag tgg atc      912
Ser Ser Gln Gly Asn Ser Val Tyr Gln Ile Glu Met Ile Gln Trp Ile
    290                 295                 300

aag cag acc tac ccg cag atc gac gtt gtc gcc ggt aac gtc gtc aca      960
Lys Gln Thr Tyr Pro Gln Ile Asp Val Val Ala Gly Asn Val Val Thr
305                 310                 315                 320

cga gag cag gct gcc agc ctg atc gcc gct ggt gct gac gcc ctt cgt     1008
Arg Glu Gln Ala Ala Ser Leu Ile Ala Ala Gly Ala Asp Ala Leu Arg
                325                 330                 335

gtc ggc atg ggt tcc ggt tcg atc tgc atc acc cag gaa gtg atg gct     1056
Val Gly Met Gly Ser Gly Ser Ile Cys Ile Thr Gln Glu Val Met Ala
            340                 345                 350
```

gtc ggt cga cct cag ggt acc gcc gtc cac gcc gtt gct gag ttc gcc 1104
Val Gly Arg Pro Gln Gly Thr Ala Val His Ala Val Ala Glu Phe Ala
355 360 365

tcc aag ttt ggc gtc ccc gtc atc gcc gat ggt gga att tcc aat gtc 1152
Ser Lys Phe Gly Val Pro Val Ile Ala Asp Gly Gly Ile Ser Asn Val
370 375 380

ggt cac atc gcc aaa gct ctc gca ctc ggt gct tcc gcc gtc atg atg 1200
Gly His Ile Ala Lys Ala Leu Ala Leu Gly Ala Ser Ala Val Met Met
385 390 395 400

gga ggc ttg ctc gcc gga acc aac gaa tcc ccc ggt gac tac ttc tat 1248
Gly Gly Leu Leu Ala Gly Thr Asn Glu Ser Pro Gly Asp Tyr Phe Tyr
405 410 415

cgc gac ggt aag cgt ctc aag ggt tac cgt ggt atg gga tcc atc gaa 1296
Arg Asp Gly Lys Arg Leu Lys Gly Tyr Arg Gly Met Gly Ser Ile Glu
420 425 430

gcc atg gag cac cag aag aag ggc aag atc gcc ggc gcc acc ggt aaa 1344
Ala Met Glu His Gln Lys Lys Gly Lys Ile Ala Gly Ala Thr Gly Lys
435 440 445

ggt gct gcc aag gct gac aag gtt gct acc gac gaa aac gcc gct acg 1392
Gly Ala Ala Lys Ala Asp Lys Val Ala Thr Asp Glu Asn Ala Ala Thr
450 455 460

cag cga tac ttt tct gaa agc gac gcc gtc aag gtc gcc cag ggc gtt 1440
Gln Arg Tyr Phe Ser Glu Ser Asp Ala Val Lys Val Ala Gln Gly Val
465 470 475 480

gca ggt gct gtg cag gac aag ggc tcg gtc aag aag ttc ttg cct tac 1488
Ala Gly Ala Val Gln Asp Lys Gly Ser Val Lys Lys Phe Leu Pro Tyr
485 490 495

ctg tac acc ggt ctg caa cac tcg ttg cag gac atg ggt gtc cca cac 1536
Leu Tyr Thr Gly Leu Gln His Ser Leu Gln Asp Met Gly Val Pro His
500 505 510

ctc tac cag ttg cgc tct gca gtg gcc tcg ggc cag gtg agg ttc gag 1584
Leu Tyr Gln Leu Arg Ser Ala Val Ala Ser Gly Gln Val Arg Phe Glu
515 520 525

ttg agg acc gca agc gcc cag gtc gag ggt ggt gtc cac ggg ctt cac 1632
Leu Arg Thr Ala Ser Ala Gln Val Glu Gly Gly Val His Gly Leu His
530 535 540

agc tac gag aag cgt ctg ttc tct tcg tag 1662
Ser Tyr Glu Lys Arg Leu Phe Ser Ser
545 550

<210> 4
<211> 553
<212> PRT
<213> Ustilago maydis

<400> 4

Met Pro Ala Ser Asn Gly Ile Gln Leu Pro Gln Asp Glu Ala Val Leu
1 5 10 15

Ser Pro Ser Gln Ala Leu Glu His Leu Lys Thr Tyr Thr Tyr Gly Asp
20 25 30

Gly Leu Ser Met Ala Glu Leu Ile Asp Ser Arg Gln His Gly Gly Leu
35 40 45

Thr Tyr Asn Asp Phe Leu Val Leu Pro Gly Phe Ile Asn Phe Ala Ala
50 55 60

Ser Asp Val Ser Leu Arg Thr Lys Val Thr Lys Asn Val Thr Leu Asn
65 70 75 80

Thr Pro Phe Leu Ser Ser Pro Met Asp Thr Val Thr Glu Thr Glu Met
85 90 95

Ala Ile Ala Met Gly Leu Met Gly Gly Met Gly Val Ile His Asn Asn
100 105 110

Met Ser Pro Gln Glu Gln Ala Ser Val Val Arg Lys Val Lys Lys Tyr
115 120 125

Glu Asn Gly Phe Ile Thr Glu Pro Leu Cys Leu Asp Pro Lys Ala Thr
130 135 140

Val Gly Asp Val Leu Asp Val Lys Glu Arg Leu Gly Phe Gly Gly Ile
145 150 155 160

Pro Ile Thr Asp Thr Gly Ala Met His Gly Lys Leu Leu Gly Ile Val
165 170 175

Thr Ala Arg Asp Val Gln Phe Arg Asp Thr Thr Leu Pro Leu Ser Glu
180 185 190

Val Met Thr Thr Asp Leu Val Thr Ala Lys Gln Gly Val Thr Leu Glu
195 200 205

Gln Ala Asn Thr Ile Leu Arg Asp Ser Lys Lys Gly Lys Leu Pro Ile
210 215 220

Val Asp Ala Glu Gly Arg Leu Val Ala Leu Leu Ala Arg Ser Asp Leu
225 230 235 240

Leu Lys Asn Gln Asn Phe Pro Leu Ala Ser Lys Arg Pro Glu Ser Lys
245 250 255

Gln Leu Tyr Cys Ala Ala Ala Ile Gly Thr Arg Pro Ser Asp Arg Glu
260 265 270

Arg Leu Ser Leu Leu Val Glu Ala Gly Leu Asp Val Val Ile Leu Asp
275 280 285

Ser Ser Gln Gly Asn Ser Val Tyr Gln Ile Glu Met Ile Gln Trp Ile
290 295 300

Lys Gln Thr Tyr Pro Gln Ile Asp Val Val Ala Gly Asn Val Val Thr
305 310 315 320

Arg Glu Gln Ala Ala Ser Leu Ile Ala Ala Gly Ala Asp Ala Leu Arg
325 330 335

Val Gly Met Gly Ser Gly Ser Ile Cys Ile Thr Gln Glu Val Met Ala
340 345 350

Val Gly Arg Pro Gln Gly Thr Ala Val His Ala Val Ala Glu Phe Ala
355 360 365

Ser Lys Phe Gly Val Pro Val Ile Ala Asp Gly Gly Ile Ser Asn Val
370 375 380

Gly His Ile Ala Lys Ala Leu Ala Leu Gly Ala Ser Ala Val Met Met
385 390 395 400

Gly Gly Leu Leu Ala Gly Thr Asn Glu Ser Pro Gly Asp Tyr Phe Tyr
405 410 415

Arg Asp Gly Lys Arg Leu Lys Gly Tyr Arg Gly Met Gly Ser Ile Glu
420 425 430

Ala Met Glu His Gln Lys Lys Gly Lys Ile Ala Gly Ala Thr Gly Lys
435 440 445

Gly Ala Ala Lys Ala Asp Lys Val Ala Thr Asp Glu Asn Ala Ala Thr
450 455 460

Gln Arg Tyr Phe Ser Glu Ser Asp Ala Val Lys Val Ala Gln Gly Val
465 470 475 480

Ala Gly Ala Val Gln Asp Lys Gly Ser Val Lys Lys Phe Leu Pro Tyr
485 490 495

Leu Tyr Thr Gly Leu Gln His Ser Leu Gln Asp Met Gly Val Pro His
500 505 510

Leu Tyr Gln Leu Arg Ser Ala Val Ala Ser Gly Gln Val Arg Phe Glu
515 520 525

Leu Arg Thr Ala Ser Ala Gln Val Glu Gly Gly Val His Gly Leu His
530 535 540

Ser Tyr Glu Lys Arg Leu Phe Ser Ser
545 550

<210> 5
<211> 26
<212> DNA
<213> artificial sequence

<400> 5

agatatccct gctagcaacg gtattc 26

<210> 6
<211> 28
<212> DNA
<213> artificial sequence

<400> 6

cgcagacaag agaagcatcc gccggcga 28

## Beschreibung

Die Erfindung bezieht sich auf Polypeptide aus pflanzenpathogenen Pilzen mit der biologischen Aktivität einer Inosinmonophosphat Dehydrogenase, dafür kodierende Nukleinsäuren, die Verwendung der Polypeptide und Nukleinsäuren zum Identifizieren von Modulatoren der Polypeptide, Verfahren zum Identifizieren solcher Modulatoren und die Verwendung dieser Modulatoren als Fungizide.

Unerwünschtes Pilzwachstum, das in der Landwirtschaft jedes Jahr zu beträchtlichen Schäden führt, kann durch die Verwendung von Fungiziden kontrolliert werden. Die Ansprüche an Fungizide sind dabei hinsichtlich ihrer Wirksamkeit, Kosten und vor allem ihrer Umweltverträglichkeit stetig angestiegen. Es existiert deshalb ein Bedarf an neuen Substanzen bzw. Substanzklassen, die zu leistungsfähigen und umweltverträglichen neuen Fungiziden entwikkelt werden können. Im Allgemeinen ist es üblich, in Gewächshaustests nach solchen neuen Leitstrukturen zu suchen. Solche Tests sind jedoch arbeitsintensiv und teuer. Die Anzahl der Substanzen, die im Gewächshaus getestet werden können, ist entsprechend begrenzt. Eine Alternative zu solchen Tests ist die Verwendung von sogenannten Hochdurchsatzverfahren (HTS = high throughput screening). Dabei werden in einem automatisierten Verfahren eine große Anzahl von Einzelsubstanzen hinsichtlich ihrer Wirkung auf Zellen, individuelle Genprodukte oder Gene getestet. Wird für bestimmte Substanzen eine Wirkung nachgewiesen, so können diese in herkömmlichen Screeningverfahren untersucht und gegebenenfalls weiter entwickelt werden.

Vorteilhafte Angriffspunkte für Fungizide werden oft in essentiellen Biosynthesewegen gesucht. Ideale Fungizide sind weiterhin solche Stoffe, die Genprodukte hemmen, die eine entscheidende Bedeutung bei der Ausprägung der Pathogenität eines Pilzes haben.

Aufgabe der vorliegenden Erfindung war es deshalb, einen geeigneten neuen Angriffspunkt für potentielle fungizide Wirkstoffe zu identifizieren und zugänglich zu machen, und ein Verfahren zur Verfügung zu stellen, das die Identifizierung von Modulatoren dieses Angriffspunkts ermöglicht, die als Fungizide verwendet werden können.

Die Inosinmonophosphat Dehydrogenase (EC 1.1.1.205), im Folgenden mit IMP Dehydrogenase oder IMPDH abgekürzt, katalysiert den geschwindigkeitslimitierenden und irreversiblen Schritt der *de novo* GTP-Synthese. Dabei wird Inosin-5'-monophosphat (IMP) mit Nicotinamid Adenin Dinucleotid ($NAD^+$) durch die IMPDH zu NADH/$H^+$ und XMP umgesetzt (Abb. 1). Inosin-5'-monophosphat ist das Endprodukt der *de novo* Purin-Biosynthese und ein essentielles Intermediat bei der oben genannten Synthese von Adenin- und Guaninnukleotiden. Die IMPDH ist somit ein Schlüsselenzym zur Bereitstellung des GTP, welches von immenser Bedeutung für die Zelle ist. GTP wird in RNA und DNA eingebaut, dient als Substrat für GTPasen (z.B. in Signaltransduktionswegen der Zelle wie G-Protein gekoppelte Rezeptoren), für die Guanylat-Zyklase (Herstellung des sekundären Botenstoffs cGMP) sowie als Co-Substrat bei vielen enzymatischen Reaktionen und spielt bei der posttranskriptionalen Modifikation der mRNA (5'-Cap-Struktur) eine Rolle. Die IMPDH ist deshalb bereits seit geraumer Zeit ein Angriffspunkt für die Suche nach antiviralen, immunsuppressiven, cancerostatischen und antimikrobiellen Wirkstoffen.

Daneben gibt es Hinweise darauf, dass die IMPDH in Pflanzen noch eine völlig andere Rolle spielt, die mit der Assimilation, dem Transport und der Speicherung von Stickstoff in Verbindung steht. So gibt es Hinweise darauf, dass die IMPDH bei der Bildung von Ureiden und Allantoin beteiligt ist.

Auch aus bestimmten Pilzen sind bereits Polypeptide mit der Aktivität einer IMPDH bekannt geworden. Dazu gehört die IMPDH aus *Candida albicans,* einem humanpathogenen Pilz, deren Sequenz 521 Aminosäuren umfasst. Die zugehörige Sequenz kann z.B. über die so genannte "trembl" Datenbank (U85049, AF249293) erhalten werden. Ebenso zugänglich sind die Sequenzen aus den häufig für Fermentationen eingesetzten filamentösen Pilzen *Ashbya gossypii* mit 522 Aminosäuren (trembl, A94860 bzw. EP 0 927 761 A2), *Saccharomyces pombe* mit 524 AS (Z97211) und *Saccharomyces cerevisiae* mit 524 Aminosäuren (Z46729) sowie dem einzelligen, humanpathogenen Pilz *Pneumocystis carinii* mit 529 Aminosäuren (AF196975). Interessanterweise sind aus der Hefe bereits mehrere für ein Polypeptid mit der Aktivität einer IMPDH kodierenden Gene identifiziert worden.

Die Mycophenolsäure, ein bekannter spezifischer Inhibitor der IMPDH, ist ein bekanntes Medikament zur Unterdrückung der Immunreaktion z.B. nach Organverpflanzungen. Die Struktur einer IMPDH zusammen mit dem Inhibitor Mycophenolsäure konnte bereits aufgeklärt werden (Sintack et al. (1996) "Structure and Mechanism of Inosine Monophosphate Dehydrogenase in Complex with the Immunosuppressant Mycophenolic Acid". *Cell* 86, 921), ebenso gibt es Arbeiten zum biochemischen Mechanismus der Inhibiton der IMPDH durch die Mycophenolsäure (Fleming et al. (1996) "Inhibition of IMPDH by Mycophenolic Acid: Dissection of Forward and Reverse Pathways Using Capillary Electrophoresis". *Biochemistry* 35, 6990).

Die Mycophenolsäure zeigt eine sehr gute Wirkung gegen die IMPDH aus Säugern, ist jedoch als antimikrobieller Wirkstoff deutlich geringer wirksam (Digits et al. (1999) "Species Specific Inhibition of Inosine 5'-Monophosphate Dehydrogenase by Mycophenolic Acid." *Biochemistry,* 38,15388). Die Mycophenolsäure bindet in die Nikotinamid-Bindetasche der $NAD^+$-Bindungsstelle an das Intermediat, das bei der Bildung von XMP aus IMP entsteht. Durch die Unterschiede der aus verschiedenen Spezies stammenden Polypeptide ergibt sich jedoch eine 20 bis 450-fach stärkere Sensitivität einer menschlichen gegenüber einer mikrobiellen IMPDH (siehe dazu auch Zhang et al. (1999) "Differential Signatures of Bacterial and Mammalian IMP Dehydrogenase Enzymes." *Current Medicinal Chemistry* 6, 537).

Weitere Inhibtioren der IMPDH, die im Bereich der medizinischen Therapie bekannt sind, sind z.B. das Ribovirin, ein Analoges des Guanosins, das antivirale Eigenschaften besitzt, Tiazofurin, ein C-Nukleosid, das nach der Phosphorylierung der 5'-Hydroxylgruppe ein NAD-ähnliches Tiazofurin-Adenin-Dinukleotid bildet und als Cancerostatikum dient, oder das Mizoribine, das in der Transplantationsmedizin zur Immunosuppression eingesetzt wird (Goldstein und Colby (1999) "IMP Dehydrogenase: Structural Aspects of Inhibitor Binding". *Current Medicinal Chemistry* 6, 519). Mizoribin zeigt dagegen gegenüber dem humanpathogenen Pilz *C. albicans* kaum eine Wirkung und ist als Wirkstoff gegen Candidiasis offensichtlich überhaupt nicht verwendbar (Ishikawa (1999) "Mizoribine and Mycophenolate Mofetil". *Current Medicinal Chemistry* 6, 575)

Die aus *C. albicans* isolierte Sequenz der IMPDH zeigt deutliche Sequenzähnlichkeit zur IMPDH aus *S. cerevisiae* und anderen Organismen. Der ORF ("open reading frame", offener Leserahmen) des *C. albicans* Gens wird durch ein kleines Intron (289 Basenpaare (bp)) unterbrochen, das typische Exon-Intron Grenzen aufweist (Kohler et al. (1997) Overexpression of a cloned IMP dehydrogenase gene of *Candida albicans* confers resistance to the specific inhibitor mycophenolic acid. *J. Bacteriol*. 179, 2331). Das Wachstum von *C. albicans* Zellen kann durch 1µg/ml Mycophenolsäure inhibiert werden, während solche Zellen, die ein Plasmid mit rekombinantem IMPDH besitzen, gegen bis zu 40µg/ml resistent sind.

Im Falle von *Pneumocystis carinii* konnte gezeigt werden, dass die Mycophenolsäure als Inhibitor der IMPDH aus diesem Organismus wirken kann (O'Gara et al. (1997) "IMP dehydrogenase from *Pneumocystis carinii* as a potential target". *Antimicrob. Agents Chemother.* 41, 40) und die Nutzung der IMPDH als sensitives Target dieses Organismus wurde vorgeschlagen. Die IMPDH von *Pneumocystis carinii* (Aminosäuresequenz) zeigt eine Homolgie zu bakterieller IMPDH (31 bis 38 %), IMPDH aus Protozoen (48 bis 59 %), Säugern (60 bis 62 %) und Pilzen (62 %). Die Aktivität der rekombinanten IMPDH konnte bei einer Konzentration von 25µM Mycophenolsäure zu 50 % inhibiert werden.

Frühere Arbeiten zeigten jedoch, dass zwar wie oben beschrieben die Mycophenolsäure *in vitro* das Wachstum einiger humanpathogener Pilze hemmt (z.B. *C. albicans, Cryptococcus neoformans*), gleichzeitig zeigte die Verbindung aber keine Wirkung gegen *Candida guilliermondii, Candida krusei, Candida pseudotropicalis, Hansenula anomala* und auch nicht gegen *S. cerevisiae* (Noto et al. (1969) "Biological properties of mycophenolic acid". J. Antibiot. (Tokyo), 22, 165).

Diese Ergebnisse zeigen, dass die IMPDH aus Säugern mit dem bekannten Inhibitor Mycophenolsäure gut inhibiert werden kann, während die Wirkung des Inhibitors bei Mikroorganismen schwach ist. Bei den untersuchten humanpathogenen Pilzen scheint die Wirkung zu schwanken. Einige Pilze, wie z.B. *S. cerevisiae*, werden als nicht sensitiv gegenüber einer Inhibition durch einen IMPDH-Inhibitor beschrieben, bei anderen, humanpathogenen Pilzen kann eine Wirkung festgestellt werden, wobei eher eine fungistatische als eine fungizide Wirkung beobachtet werden kann. Die Eignung der IMPDH als Zielprotein für Fungizide, bzw. zur Verwendung für die Suche nach fungiziden Wirkstoffen ist demnach fraglich. Insbesondere kann bislang eine zumindest fungistatische Wirkung nur bei einigen humanpathogenen Pilzen gezeigt werden, während aus pflanzenpathogenen Pilzen bislang keine Nukleinsäure- oder Aminosäuresequenz einer IMPDH zur Verfügung steht, und auch keine Erkenntnisse darüber vorliegen, ob unter Umständen pflanzenpathogene Pilze gegenüber einem Inhibitor der IMPDH sensitiv reagieren, so dass solche Inhibitoren als Fungizide verwendet werden könnten.

Aufgabe der vorliegenden Erfindung war es deshalb im Besonderen, eine IMPDH eines pflanzenpathogenen Pilzes sowie Verfahren zugänglich zu machen, die geeignet sind, Inhibitoren des Enzyms zu identifizieren, sowie zu prüfen, ob solche Inhibitoren als Fungizide bei pflanzenpathogenen Pilzen verwendet werden können.

**Beschreibung der Abbildungen und des Sequenzprotokolls**

Abbildung 1: Die von der IMPDH katalysierte Reaktion ist die Umsetzung von Inosin-5'-monophosphat (IMP) zu Xanthosin-5'-monophosphat (XMP), wobei aus $NAD^+$ das NADH entsteht. Diese von der IMPDH katalysierte Reaktion beschreibt ihre biologische bzw. enzymatische Aktivität.

Abbildung 2: Die Reinigung der IMPDH aus *U. maydis* kann mit Hilfe einer Auftrennung auf einem Gel verfolgt werden. Spur 1: 10 kD-Leiter; Spur 2: Proteinextrakt einer nicht induzierten Kultur; Spur 3: Proteinextrakt einer induzierten Kultur nach 1,5 h Inkubation; Spur 4: löslicher Überstand des *E. coli* Proteinextrakts; Spur 5: Säulendurchfluss der Ni-NTA Säulen; Spur 6: Eluat 1 der Ni-NTA-Säule; Spur 7: Eluat 2 der Ni-NTA-Säule; Spur 8: 10 kD-Leiter.

Abbildung 3: Kinetik der Umsetzung von $NAD^+$ durch die IMPDH. In einem Assayvolumen von 50 µl wurden 25 µM $NAD^+$ und 50 µM IMP eingesetzt. Die verwendeten Proteinkonzentrationen sind der Abbildung zu entnehmen. Die Umsetzung wurde anhand der Fluoreszenz des entstehenden NADH verfolgt. In der Abbildung ist die relative Fluoreszenz angegeben.

Abbildung 4: $K_M$-Wert Bestimmung für $NAD^+$ mit $V_{o/max}$ = Anfangs- bzw. maximale Reaktionsgeschwindigkeit in µM/($mg^{-1}min^{-1}$)und S = Substratkonzentration in µM.

Abbildung 5: Sequenzvergleich auf Aminosäureebene zwischen der IMP Dehydrogenase aus *U. maydis*, den aus *S. cerevisiae* bekannten Sequenzen, und der IMP Dehydrogenase aus *Homo sapiens* und *A. thaliana.* Die für IMP Dehydrogenasen typische Konsensussequenz im katalytischen Zentrum des Enzyms ist fett dargestellt.

SEQ ID NO:1: Genomische DNA kodierend für IMP Dehydrogenase aus *Ustilago maydis.* Die genomische DNA enthält ein Intron.

SEQ ID NO:2: Polypeptidsequenz, kodiert von der DNA gemäß SEQ ID NO: 1.

SEQ ID NO:3: cDNA kodierend für IMP Dehydrogenase aus *Ustilago maydis.*

SEQ ID NO:4: Polypeptidsequenz, kodiert von der DNA gemäß SEQ ID NO:3.

SEQ ID NO:5: Artifizielle Sequenz (Primer), geeignet zur Gewinnung und/oder Amplifizierung der DNA gemäß SEQ ID NO: 1 und 3 sowie dazu homologer Sequenzen.

SEQ ID NO:6: Artifizielle Sequenz (Primer), geeignet zur Gewinnung und/oder Amplifizierung der DNA gemäß SEQ ID NO: 1 und 3 sowie dazu homologer Sequenzen.

**Definitionen**

Der Begriff "Identität", wie er hierin verwendet wird, bezieht sich auf die Zahl von Sequenzpositionen die in einem Alignment identisch sind. Sie wird meist in Prozent der Alignment Länge angegeben.

Der Begriff "Ähnlichkeit", wie er hierin verwendet wird, setzt dagegen die Definition einer Ähnlichkeitsmetrik voraus, also eines Maßes dafür, als wie ähnlich man beispielsweise ein Valin zu einem Threonin oder zu einem Leucin annehmen möchte.

Der Begriff "Homologie", wie er hierin verwendet wird, bedeutet wiederum evolutionäre Verwandtschaft. Zwei homologe Proteine haben sich aus einer gemeinsamen Vorläufersequenz entwickelt. Der Begriff hat nicht unbedingt etwas mit Identität oder Ähnlichkeit zu tun, abgesehen davon, dass homologe Sequenzen meist ähnlicher sind (oder in einem Alignment mehr identische Positionen besitzen) als nichthomologe Sequenzen.

Der Ausdruck "IMPDH" wie er hierin verwendet wird, steht für IMP Dehydrogenase, die alternativ auch als Inosin-5'-phosphat Dehydrogenase, Inosinat Dehydrogenase, Inosin-5'-monophosphat Dehydrogenase, Inosinmonophosphat Dehydrogenase, IMP Oxidoreduktase oder Inosinmonophosphat Oxidoreduktase bezeichnet wird, und die die Reaktion Inosin-5'-monophosphat + $NAD^+$ + $H_2O$ = Xanthosin-5'-monophosphat + $NADH/H^+$ katalysiert.

Der Ausdruck "vollständige IMPDH" wie er hierin verwendet wird, beschreibt die IMPDH, die von der vollständigen kodierenden Region einer Transkriptionseinheit kodiert wird, beginnend mit dem ATG-Startcodon und umfassend alle informationstragenden Exonbereiche des im Herkunftsorganismus vorliegenden, für IMPDH kodierenden Gens, sowie die für eine korrekte Termination der Transkription nötigen Signale.

Der Ausdruck "biologische Aktivität einer IMPDH" wie er hierin verwendet wird, bezieht sich auf die Fähigkeit eines Polypeptids, die vorstehend beschriebene Reaktion, d.h. die Umsetzung von IMP zu XMP unter gleichzeitiger Umsetzung von $NAD^+$ zu NADH zu katalysieren.

Der Ausdruck "aktives Fragment" wie er hierin verwendet wird, beschreibt nicht mehr vollständige Nukleinsäuren kodierend für IMPDH, die aber noch für Polypeptide mit der biologischen Aktivität einer IMPDH kodieren, und die eine für die IMPDH charakteristische Reaktion wie vorne beschrieben katalysieren können. Solche Fragmente sind kürzer als die oben beschriebenen vollständigen, für die IMPDH kodierenden Nukleinsäuren. Dabei können sowohl an den 3'- und/oder 5'-Enden der Sequenz Nukleinsäuren entfernt worden sein, es können aber auch Teile der Sequenz deletiert, d.h. entfernt worden sein, die die biologische Aktivität der IMPDH nicht entscheidend beeinträchtigen. Eine geringere oder gegebenenfalls auch eine erhöhte Aktivität, die aber noch die Charakterisierung bzw. Verwendung des resultierenden IMPDH Fragments gestattet, wird dabei als ausreichend im Sinne des hier verwendeten Ausdrucks verstanden. Der Ausdruck "aktives Fragment" kann sich ebenso auf die Aminosäuresequenz der IMPDH beziehen und gilt dann analog den obigen Ausführungen für solche Polypeptide, die im Vergleich zur oben definierten vollständigen Sequenz bestimmte Teile nicht mehr enthalten, wobei die biologische Aktivität des Enzyms jedoch nicht entscheidend beeinträchtigt ist. Die Fragmente können dabei verschiedene Länge besitzen, z.B. mindestens 120, mindestens 300, mindestens 400 oder mindestens 500 Aminosäuren.

Der Ausdruck "Gen", wie er hierin verwendet wird, ist die Bezeichnung für einen Abschnitt aus dem Genom einer Zelle, die für die Synthese einer Polypeptid-Kette verantwortlich ist.

Der Ausdruck "cDNA" wie er hierin verwendet wird, beschreibt komplementäre, von einer mRNA durch reverse Transkription erhaltene DNA. Sie enthält nur die den Exons der genomischen DNA entsprechenden Sequenzen. Nach cDNA-Sequenzierung läßt sich gegebenenfalls die Aminosäuresequenz des davon kodierten Proteins ableiten. Nach Einführung einer cDNA in eine Zelle können von dem jeweiligen davon kodierten Protein große Mengen synthetisiert werden.

Der Ausdruck "hybridisieren", wie er hierin verwendet wird, beschreibt den Vorgang, bei welchem ein einzelsträngiges Nukleinsäuremolekül mit einem komplementären Strang eine Basenpaarung eingeht. Auf diese Weise können z.B. ausgehend von der hierin genannten oder ableitbaren Sequenzinformation beispielsweise DNA-Fragmente aus anderen phytopathogenen Pilzen als *Ustilago maydis* isoliert werden, welche für IMPDHs kodieren, welche dieselben oder ähnliche Eigenschaften einer der erfindungsgemäße IMPDH aufweisen.

Hybridisierungsbedingungen werden nach folgender Formel näherungsweise berechnet:

Die Schmelztemperatur

$$Tm = 81{,}5°C + 16{,}6\ \{\log[c(Na^+)]\} + 0{,}41(\%\ G + C) - (500/n)$$

(Lottspeich, F., Zorbas H. (Hrsg.). (1998). Bioanalytik. Spektrum Akademischer Verlag, Heidelberg, Berlin).

Dabei ist c die Konzentration und n die Länge des hybridisierenden Sequenzabschnitts in Basenpaaren. Für eine Sequenz >100 bp entfällt der Ausdruck 500/n. Mit höchster Stringenz wird bei einer Temperatur 5-15°C unterhalb Tm und einer Ionenstärke von 15 mM $Na^+$ (entspricht 0.1 x SSC) gewaschen. Wird eine RNA-Probe zur Hybridisierung verwendet, so ist der Schmelzpunkt um 10-15°C höher.

Bevorzugte Hybridisierungsbedingungen sind nachstehend angegeben:

Hybridisierungslösung: DIG Easy Hyb (Roche, ZZ) Hybridisierungstemperatur: 42°C bis 70°C, bevorzugt bei 42-65°C (DNA-DNA) oder 50°C (DNA-RNA). Im vorliegenden Fall besonders geeignete stringente Temperaturen für die Hybridisierung liegen zwischen 50 und 65°C, wobei eine Temperatur von 65°C eine insbesondere geeignete stringente Temperatur darstellt.

1. Waschschritt: 2 x SSC, 0,1 % SDS 2 x 5 min bei Raumtemperatur;
2. Waschschritt: 1 x SSC, 0,1 % SDS 2 x 15 min bei 50°C; bevorzugt 0,5 x SSC, 0,1 % SDS 2 x 15 min bei 65°C; besonders bevorzugt 0,2 x SSC, 2 x 15 min bei 68°C.

Der Grad der Identität der Nukleinsäuren wird vorzugsweise bestimmt mit Hilfe des Programms NCBI BLASTN Version 2.0.4. (Altschul et al. (1997) "Gapped BLAST and PSI-BLAST: a new generation of protein database search programs", *Nucleic Acids Res.* 25:3389).

Der Ausdruck "Fungizid" bzw. "fungizid" wie er hierin verwendet wird, bezieht sich auf chemische Verbindungen, die zur Bekämpfung solcher Pilze geeignet sind, die Pflanzen, Pflanzenteile oder Pflanzenprodukte befallen und schädigen oder deren Ertrag bzw. Wert mindern. Zu den genannten Pflanzenteilen gehören beispielsweise Blätter, Samen und Früchte (wie z.B. Beeren, Obst, Getreidekörner). Zu den genannten Pflanzenprodukten gehören aus den Pflanzen gewonnene Rohstoffe oder Substanzen wie beispielsweise Hölzer oder Fasern.

Der Ausdruck "heterologer Promotor", wie er hierin verwendet wird, bezieht sich auf einen Promotor, der andere Eigenschaften als derjenige Promotor aufweist, der im Ursprungsorganismus die Expression des betreffenden Gens kontrolliert.

Der Ausdruck "Kompetitor" wie er hierin verwendet wird, bezieht sich auf die Eigenschaft der Verbindungen, mit anderen, gegebenenfalls zu identifizierenden Verbindungen um die Bindung an der IMPDH zu kompetitieren und diese vom Enzym zu verdrängen bzw. von dieser verdrängt zu werden.

Der Ausdruck "Agonist", wie er hierin verwendet wird, bezieht sich auf ein Molekül, das die Aktivität der IMPDH beschleunigt oder verstärkt.

Der Ausdruck "Antagonist", wie er hierin verwendet wird, bezieht sich auf ein Molekül, das die Aktivität der IMPDH verlangsamt oder verhindert.

Der Ausdruck "Modulator", wie er hierin verwendet wird, stellt den Oberbegriff zu Agonist bzw. Antagonist dar. Modulatoren können kleine organisch-chemische Moleküle, Peptide oder Antikörper sein, die an die erfindungsgemäßen Polypeptide binden bzw. die deren Aktivität beeinflussen. Weiterhin können Modulatoren kleine organisch-chemische Moleküle, Peptide oder Antikörper sein, die an ein Molekül binden, welches wiederum an die erfindungsgemäßen Polypeptide bindet, und dadurch deren biologische Aktivität beeinflusst. Modulatoren können natürliche Substrate und Liganden darstellen oder strukturelle oder funktionelle Mimetika davon. Bevorzugt handelt es sich beim Ausdruck "Modulator", wie er hierin verwendet wird, jedoch um solche Moleküle, die nicht die natürlichen Substrate bzw. Liganden darstellen.

Der Ausdruck "Inhibitor" bzw. "spezifischer Inhibitor", wie er hierin gebraucht wird, bezeichnet eine Substanz, die direkt oder indirekt zumindest eine, gegebenenfalls aber auch mehrere der vorstehend genannten enzymatischen Aktivitäten inhibiert. Ein solcher Inhibitor ist vorzugsweise spezifisch, d.h. er inhibiert die IMPDH-Aktivität bei einer Konzentration die niedriger ist als die Konzentration eines Inhibitors, die benötigt wird, um einen anderen, damit nicht verbundenen Effekt hervorzurufen. Vorzugsweise ist die Konzentration zweifach niedriger, insbesondere bevorzugt fünffach niedriger und ganz besonders bevorzugt zumindest zehnfach oder 20fach niedriger als die Konzentration einer Verbindung, die zum Hervorrufen eines unspezifischen Effekts benötigt wird.

Im Rahmen der vorliegenden Erfindung wurde die Nukleinsäuresequenz kodierend für die IMPDH aus dem pflanzenpathogenen Pilz *U. maydis* identifiziert und isoliert und das davon kodierte Polypeptid gewonnen. Weiterhin wurde ein Verfahren entwickelt, das geeignet ist, die Aktivität der IMPDH zu bestimmen sowie Inhibitoren des Enzyms auch in HTS- und UHTS-Verfahren zu identifizieren und zu bewerten. Im Rahmen der vorliegenden Erfindung wurde weiter gezeigt, dass die Inhibtoren der IMPDH aus pflanzenpathogenen Pilzen als Pflanzenschutzmittel verwendet werden können.

Der Brandpilz *Ustilago maydis*, ein Basidiomycet, befällt Maispflanzen. Die Krankheit kommt in allen Maisanbaugebieten vor, erreicht jedoch nur in trockenen Jahren eine größere Bedeutung. Typische Symptome sind die beulenartigen, faustgroßen Anschwellungen (Brandbeulen), die an allen oberirdischen Pflanzenteilen gebildet werden. Die Beulen sind zuerst von einer weiss-grauen, derben Haut überzogen. Beim Aufreissen der Haut wird eine schwarze, zunächst schmierige, später pulvrige Brandsporenmasse frei. Weitere Arten der Gattung Ustilago sind z. B. *U. nuda* (verursacht Gersten- und Weizenflugbrand), *U. nigra* (verursacht Gerstenschwarzbrand), *U. hordei* (verursacht Gerstenhartbrand) und *U. avenae* (verursacht Haferflugbrand).

Die IMPDH aus *U. maydis* (im Folgenden als "Imp1" bezeichnet) erstreckt sich über 1999 bp. Die kodierende cDNA ist 1659 bp lang. *Imp1* enthält demnach ein Intron von 340 bp Länge. Das aus dem ORF der cDNA abgeleitete Polypeptid ist 553 Aminosäuren lang. Dies entspricht einem errechneten Molekulargewicht von etwa 60 kD für das Monomer.

Die für Imp1 kodierende Nukleinsäuresequenz ist als SEQ ID NO:1 (geonomische Sequenz) bzw. SEQ ID NO: 3 (cDNA) in der vorliegenden Anmeldung offenbart. Die jeweils davon kodierten Polypeptide sind in der vorliegenden Anmeldung als SEQ ID NO: 2 und SEQ ID NO: 4 abgelegt.

In der vorliegenden Erfindung wird damit zum ersten Mal eine IMPDH eines phytopathogenen Pilzes, hier *Ustilago maydis*, vollständig beschrieben. Neben der IMPDH aus *Saccharomyces cerevisiae, Saccharomyces pombe, Candida albicans, C. neoformans* und *Pneumocystis carinii,* die vorstehend beschrieben wurden, sind bislang keine Sequenzen anderer Pilze, die für eine IMPDH kodieren, bekannt geworden. Insbesondere die für eine IMPDH kodierende Sequenz aus einem pflanzenpathogenen Basidiomyceten war bislang noch nicht bekannt.

Ein Homologievergleich der Sequenz aus *U. maydis* kodierend für IMPDH gemäß SEQ ID NO: 1 bzw. 3 mit bekannten für IMPDH kodierenden Sequenzen aus anderen Pilzen, der humanen Sequenz sowie der Sequenz aus *A. thaliana* ergibt, dass die Sequenz aus *S. cerevisiae* die größte Homologie zur Sequenz aus *U. maydis* aufweist (Tab. I). Dies ist insbesondere deshalb von Interesse, als die vorstehend beschriebene Mycophenolsäure, ein bekannter Inhibitor der humanen IMPDH, der bei *C. albicans* noch fungistatisch wirkt, keine Wirkung bei *S. cerevisiae* zeigt (Noto et al. (1969) "Biological properties of mycophenolic acid". J. Antibiot. (Tokyo), 22, 165). Tabelle I listet vergleichend die Homologie von DNA kodierend für IMPDH aus verschiedenen Organismen zur DNA kodierend für IMPDH aus *U. maydis* auf.

Tabelle I

| Organismus | Homologie in % |
|---|---|
| *P. carinii* | 59 |
| *C. neoformans* | 55 |
| *C. albicans* | 59 |
| *S. cerevisiae* (*imh1*) | 60 |
| *S. cerevisiae* (*imh2*) | 63 |

Tabelle I (fortgesetzt)

| Organismus | Homologie in % |
|---|---|
| *S. cerevisiae* (*imh3*) | 64 |
| *H sapiens* | 59 |
| *A. thaliana* | 43 |

Im Rahmen der vorliegenden Erfindung wurde mittels Knockout-Analysen im Basidiomyceten *Ustilago maydis* überraschend festgestellt, dass das Enzym in diesem pflanzenpathogenen Pilz wichtig zum Überleben des Organismus ist. Daraus kann geschlossen werden, dass die IMPDH für pflanzenpathogene Pilze bzw. Pilze im Allgemeinen eine besondere Rolle spielt. Entsprechende Ergebnisse konnten bislang in Pilzen jedoch nicht gezeigt werden, auch nicht in *S. cerevisiae*, was unter Umständen darauf zurückzuführen ist, dass in Hefe mehrere für IMPDH kodierende Gene vorliegen und die Rolle der IMPDH deshalb nicht erkannt wurde. Die IMPDH wurde damit erstmals als ein optimales Target für die Suche nach neuen, spezifischen Fungiziden gegen pflanzenpathogene Pilze erkannt. Damit ist die Möglichkeit gegeben, mit Hilfe dieses Targets gegebenenfalls völlig neue Leitstrukturen zu identifizieren, die die IMPDH inhibieren und als Pflanzenschutzmittel bzw. Fungizide verwendet werden können.

Im Rahmen der vorliegenden Erfindung wurde weiter gefunden, dass die IMPDH zum Identifizieren von Substanzen in geeigneten Testverfahren verwendet werden kann, die die Aktivität des Enzyms beeinflussen, was bei verschiedenen theoretisch interessanten Targets nicht selbstverständlich gegeben ist. Neben einer IMPDH aus einem phytopathogenen Pilz, die durch ihre Aminosäuresequenz und die dafür kodierende Nukleinsäuresequenz charakterisiert wird, werden deshalb auch geeignete Testverfahren zum Identifizieren von Modulatoren des Enzyms zur Verfügung gestellt, die auch zur Verwendung in HTS-Verfahren geeignet sind.

Im Rahmen der vorliegenden Erfindung wurde weiter gefunden, dass die IMPDH *in vitro* tatsächlich durch Wirkstoffe inhibiert werden kann und auch ein mit diesen Wirkstoffen behandelter pilzlicher Organismus durch die Behandlung mit diesen Wirkstoffen geschädigt und abgetötet werden kann. Die Inhibitoren einer IMPDH aus pflanzenpathogenen Pilzen können also als Fungizide im Pflanzenschutz verwendet werden. In der vorliegenden Erfindung wird beispielsweise gezeigt, dass die Hemmung der IMPDH mit in einem oben genannten Testverfahren identifizierten Substanzen zum Absterben der behandelten Pilze in synthetischen Medien bzw. auf der Pflanze führt.

Wie bereits vorstehend geschildert wurde, war trotz der intensiven Forschung an IMPDHs und trotz der bereits bekannten IMPDH-Inhibitoren, die in der Humanmedizin eingesetzt werden, bislang unbekannt, dass die IMPDH in pflanzenpathogenen Pilzen ein Zielprotein (ein so genanntes "Target") fungizid wirksamer Substanzen sein kann. Damit wird in der vorliegenden Erfindung zum ersten Mal gezeigt, dass die IMPDH ein insbesondere für phytopathogene Pilze wichtiges Enzym darstellt und deshalb in besonderem Maße dazu geeignet ist, als Zielprotein für die Suche nach weiteren und verbesserten fungizid wirksamen Wirkstoffen verwendet zu werden.

IMPDHs teilen sich mehrere homologe Bereiche. Eine dieser Regionen konzentriert sich auf das katalytische Zentrum, insbesondere auf ein Cystein, das eine Rolle bei der Bindung des IMP spielen dürfte. Dieses Zentrum ist ein für IMP-Dehydrogenasen charakteristisches Sequenzmotiv. Durch eine geeignete Suche in der PROSITE database konnte ein solches Motiv identifiziert werden (Hofmann K., Bucher P., Falquet L., Bairoch A. (1999) "The PROSITE database, its status in 1999". *Nucleic Acids Res.* 27, 215). Es kann wie folgt dargestellt werden:

[LIVM]-[RK]-[LIVM]-G-[LIVM]-G-x-G-S-[LIVM]-C-x-T,

PROSITE ermöglicht funktionelle Domänen von IMP-Dehydrogenasen zu identifizieren und ist geeignet, die Funktion eines Genprodukts vorherzusagen.

Bei der Darstellung des Prosite Motivs wird der "Ein-Buchstaben-Code" verwendet. Das Symbol "x" steht für eine Position, an der jede Aminosäure akzeptiert wird. Eine variable Position, an der verschiedene bestimmte Aminosäuren akzeptiert werden, wird in eckigen Klammern "[...]" dargestellt, wobei die an dieser Position möglichen Aminosäuren aufgezählt werden. Aminosäuren, die an einer bestimmten Position nicht akzeptiert werden, stehen dagegen in geschweiften Klammern "{...}". Ein Gedankenstrich "-" trennt die einzelnen Elemente bzw. Positionen des Motivs. Wiederholt sich eine bestimmte Position, z.B. "x", mehrfach hintereinander, kann dies durch Angabe der Zahl der Wiederholungen in einer nachfolgenden Klammer dargestellt werden, z.B. "x (3)", was für "x-x-x" steht.

Ein Prosite Motiv stellt also letztlich die Komponenten einer Konsensussequenz dar, sowie Abstände zwischen den beteiligten Aminosäuren und ist damit typisch für eine bestimmte Enzymklasse. Anhand dieses Motivs können auf Basis der erfindungsgemäßen Nukleinsäuren weitere Polypeptide aus pflanzenpathogenen Pilzen indentifiziert bzw. zugeordnet werden, die zur selben Klasse wie das erfindungsgemäße Polypeptid gehören.

Im Falle der IMPDH aus *U. maydis* liegt dieses Motiv ebenso vor wie bei *S. cerevisiae, Homo sapiens* oder *Arabidopsis thaliana* (siehe Abbildung 5), wobei sich die Sequenz von *U. maydis* von der von *S. cerevisiae* an einer bzw. zwei Positionen unterscheidet. Die spezifische Konsensussequenz für eine erfindungsgemäße IMP Dehydrogenase, die zur Identifizierung bzw. Zuordnung weiterer erfindungsgemäßer Polypeptide genutzt werden kann, ist deshalb besonders bevorzugt

-LRVGMGSGSICIT-.

Das oben genannte Prosite Motiv bzw. die spezifische Konsensussequenz sind typisch für die erfindungsgemäßen Polypeptide, die anhand dieser Konsensussequenzen strukturell definiert werden können und damit auch eindeutig identifizierbar sind.

Offenbart werden deshalb auch Polypeptide aus pflanzenpathogenen Pilzen mit der biologischen Aktivität einer IMPDH, die das vorstehend genannte Prosite Motiv [LIVM]-[RK]-[LIVM]-G-[LIVM]-G-x-G-S-[LIVM]-C-x-T umfassen, bevorzugt solche Polypeptide, die die vorstehend genannte Konsensussequenz -LRVGMGSGSICIT- umfassen.

Aufgrund der vorstehenden Ergebnisse und der Homologie, die bei speziesspezifischen Nukleinsäuren kodierend für IMPDH vorliegen, können auch IMPDHs aus anderen pflanzenpathogenen Pilzen identifiziert und verwendet werden, um die oben gestellte Aufgabe zu lösen, d.h. sie können ebenfalls zum Identifizieren von Inhibitoren der IMPDH verwendet werden, welche wiederum als Fungizide im Pflanzenschutz verwendet werden können. Es ist jedoch auch denkbar einen anderen Pilz, der nicht pflanzenpathogen ist, bzw. dessen IMPDH oder die dafür kodierende Sequenz zu verwenden, um fungizid wirkende Inhibitoren der IMPDH zu identifizieren. Aufgrund der hier angegebenen Sequenz gemäß SEQ ID NO: 1 oder SEQ ID NO: 3 bzw. der unter SEQ ID NO: 5 und 6 angegebenen Primer und gegebenenfalls unter Zuhilfenahme der vorstehend gezeigten Konsensussequenz bzw. des Prosite Motivs ist es dem Fachmann möglich, z.B. mittels PCR weitere für IMP Dehydrogenasen kodierende Nukleinsäuren aus anderen pflanzenpathogenen Pilzen zu erhalten und zu identifizieren. Solche Nukleinsäuren und deren Verwendung in Verfahren zum Identifizieren von fungiziden Wirkstoffen werden als von der vorliegenden Erfindung umfasst betrachtet.

Offenbart sind deshalb Nukleinsäuren, die für vollständige Polypeptide aus pflanzenpathogenen Pilzen mit der biologischen Aktivität einer IMPDH kodieren. Diese Nukleinsäuren weisen bevorzugt eine Identität von mindestens 60 %, mindestens 65 %, mindestens 70 %, mindestens 75 %, mindestens 80 %, mindestens 85 %, mindestens 90 %, mindestens 95 % und insbesondere mindestens 98 % über eine Länge von 60, 300, 600 oder 1200 Basenpaaren und bevorzugt über die Gesamtlänge der kodierenden Sequenz zueinander auf. Die genannten Nukleinsäuren umfassen jeweils bevorzugt das oben genannte Prosite Motiv oder die oben genannten Konsensussequenz.

Gegenstand der vorliegenden Erfindung sind insbesondere Nukleinsäuren, die für IMPDHs aus pflanzenpathogenen Basidiomyceten, bevorzugt aus der Gattung *Ustilago* kodieren.

Gegenstand der vorliegenden Erfindung sind ganz besonders bevorzugt Nukleinsäuren, die für IMPDH aus *Ustilago maydis* kodieren.

Gegenstand der vorliegenden Erfindung sind insbesondere bevorzugt Nukleinsäuren aus *Ustilago maydis,* die für ein Polypeptid gemäß SEQ ID NO: 2 bzw. SEQ ID NO: 4 oder aktive Fragmente davon kodieren. Insbesondere sind die Nukleinsäuren gemäß SEQ ID NO: 1 und SEQ ID NO: 3 Gegenstand der vorliegenden Erfindung.

Bei den erfindungsgemäßen Nukleinsäuren handelt es sich insbesondere um einzelsträngige oder doppelsträngige Desoxyribonukleinsäuren (DNA) oder Ribonukleinsäuren (RNA). Bevorzugte Ausführungsformen sind Fragmente genomischer DNA, die Introns enthalten können, und cDNAs.

Bevorzugt handelt es sich bei den erfindungsgemäßen Nukleinsäuren um DNA-Fragmente, die der cDNA der erfindungsgemäßen Nukleinsäuren entsprechen.

Besonders bevorzugt umfassen die erfindungsgemäßen Nukleinsäuren eine Sequenz aus phytopathogenen Pilzen kodierend für ein Polypeptid mit der biologischen Aktivität einer IMPDH ausgewählt aus

a) der Sequenz gemäß SEQ ID NO: 1 oder SEQ ID NO: 3

b) Sequenzen, die für ein Polypeptid kodieren, welches die Aminosäuresequenz gemäß SEQ ID NO: 2 oder SEQ ID NO: 4 umfasst,

Die erfindungsgemäßen Nukleinsäuren können auf die übliche Weise hergestellt werden. Beispielsweise können die Nukleinsäuremoleküle vollständig chemisch synthetisiert werden. Man kann auch kurze Stücke der erfindungsgemäßen Nukleinsäuren wie z.B. die Oligonukleotide gemäß SEQ ID NO: 5 und 6 chemisch synthetisieren und solche Oligonukleotide radioaktiv oder mit einem Fluoreszenzfarbstoff markieren. Diese markierten Oligonukleotide können auch verwendet werden, um von mRNA z.B. aus phytopathogenen Pilzen hergestellte cDNA-Banken zu durchsuchen. Klone, an die die markierten Oligonukleotide hybridisieren, werden zur Isolierung der betreffenden DNA-Fragmente ausgewählt. Nach der Charakterisierung der isolierten DNA erhält man auf einfache Weise die erfindungsgemäßen Nukleinsäuren.

Die erfindungsgemäßen Nukleinsäuren können auch mittels PCR-Verfahren unter Verwendung chemisch synthetisierter Oligonukleotide hergestellt werden.

Der Ausdruck "Oligonukleotid(e)", wie er hierin verwendet wird, bezieht sich auf DNA-Moleküle, die aus 10 bis 50 Nukleotiden, vorzugsweise 15 bis 30 Nukleotiden bestehen. Sie werden chemisch synthetisiert und können als Sonden für Hybridisierungsversuche oder als Primer für PCR (Polymerase Chain Reaction) verwendet. werden.

Zur Herstellung der erfindungsgemäßen Polypeptide, insbesondere des von der Nukleinsäuresequenz gemäß SEQ ID NO: 1 oder SEQ ID NO: 3 kodierten Polypeptids, können außerdem Wirtszellen, die mindestens eine der erfindungsgemäßen Nukleinsäuren enthalten, unter geeigneten Bedingungen kultiviert werden. Die gewünschten Polypeptide können danach auf übliche Weise aus den Zellen oder dem Kulturmedium isoliert werden. Die Polypeptide können auch in *in vitro*-Systemen hergestellt werden.

Zur Herstellung der erfindungsgemäßen IMPDH aus *Ustilago maydis* kann das Gen z.B. rekombinant in *Escherichia coli* exprimiert und aus *E. coli* Zellen eine Enzympräparation hergestellt werden.

So wurde für die Expression des von *impl* kodierten Polypeptids Imp1 der zugehörige ORF aus mRNA nach dem Fachmann bekannten Methoden über genspezifische Primer amplifiziert, die im Sequenzprotokoll als SEQ ID NO: 5 und SEQ ID NO: 6 abgelegt sind. Die entsprechende cDNA wurde in den Vektor pET32a (Novagen, ermöglicht die Einführung eines His-Tags (N- oder C-terminal) oder eines S-Tags) kloniert. Das resultierende Plasmid p830 enthält die vollständige kodierende Sequenz von *impl* in einer N-terminalen Fusion mit Thioredoxin-Tag aus dem Vektor. Das Imp1 Fusionsprotein besitzt eine berechnete Masse von 78 kD (Beispiel 1 und Abbildung 2).

Das Plasmid p830 wurde dann zur rekombinanten Expression von Imp1 in *E. coli* Zellen verwendet (Beispiel 1).

Die Ergebnisse, die im Rahmen der vorliegenden Erfindung hier zum ersten Mal gezeigt werden, gelten ebenso auch für andere Polypeptide aus pflanzenpathogenen Pilzen mit der biologischen Aktivität einer IMPDH. So können auch homologe IMPDHs aus anderen solchen Spezies wie oben ausgeführt erhalten und in erfindungsgemäßen Verfahren verwendet werden.

Besonders bevorzugt handelt es sich bei den genannten homologen Polypeptiden um solche, die zur IMPDH aus *Ustilago maydis* eine Ähnlichkeit von mindestens 60 %, mindestens 65 %, mindestens 70 %, mindestens 75 %, mindestens 80 %, mindestens 85 %, mindestens 90 %, mindestens 95 % und insbesondere mindestens 98 % über eine Länge von wenigstens 20, vorzugsweise wenigstens 25, besonders wenigstens 30 und ganz besonders bevorzugt wenigstens 100 fortlaufenden Aminosäuren und insbesondere bevorzugt über die Gesamtlänge aufweisen.

Solche zur IMPDH aus *Ustilago maydis*, insbesondere zum Polypeptid gemäß SEQ ID NO: 2 bzw. SEQ ID NO: 4 homologen Polypeptide, die zum Identifizieren von fungiziden Wirkstoffen verwendet werden können, müssen nicht vollständige pilzliche IMPDHs darstellen, sondern können auch nur Fragmente davon sein, solange sie zumindest noch die biologische Aktivität der vollständigen IMPDH aufweisen. Polypeptide, die eine gleichartige biologische Aktivität wie eine IMPDH mit einer Aminosäuresequenz gemäß SEQ ID NO: 2 oder SEQ ID NO: 4 ausüben, werden noch als erfindungsgemäß betrachtet. Als erfindungsgemäß werden vor allem noch solche Polypeptide betrachtet, die IMPDHs beispielsweise der folgenden pflanzenpathogenen Pilze entsprechen oder Fragmenten davon, die noch deren biologische Aktivität haben:

Plasmodiophoromycetes, Oomycetes, Chytridiomycetes, Zygomycetes, Ascomycetes, Basidiomycetes und Deuteromycetes, z.B.

Pythium-Arten, wie beispielsweise *Pythium ultimum,* Phytophthora-Arten, wie beispielsweise *Phytophthora infestans*, Pseudoperonospora-Arten, wie beispielsweise *Pseudoperonospora humuli* oder *Pseudoperonospora cubensis*, Plasmopara-Arten, wie beispielsweise *Plasmopara viticola*, Bremia-Arten, wie beispielsweise *Bremia lactucae*, Peronospora-Arten, wie beispielsweise *Peronospora pisi* oder *P. brassicae,* Erysiphe-Arten, wie beispielsweise *Erysiphe graminis,* Sphaerotheca-Arten, wie beispielsweise *Sphaerotheca fuliginea*, Podosphaera-Arten, wie beispielsweise *Podosphaera leucotricha*, Venturia-Arten, wie beispielsweise *Venturia inaequalis*, Pyrenophora-Arten, wie beispielsweise *Pyrenophora teres* oder *P. graminea* (Konidienform: Drechslera, Syn: Helminthosporium), Cochliobolus-Arten, wie beispielsweise *Cochliobolus sativus* (Konidienform: Drechslera, Syn: Helminthosporium), Uromyces-Arten, wie beispielsweise *Uromyces appendiculatus*, Puccinia-Arten, wie beispielsweise *Puccinia recondita*, Sclerotinia-Arten, wie beispielsweise *Sclerotinia sclerotiorum*, Tilletia-Arten, wie beispielsweise *Tilletia caries*; Ustilago-Arten, wie beispielsweise *Ustilago nuda* oder *Ustilago avenae*, Pellicularia-Arten, wie beispielsweise *Pellicularia sasakii*, Pyricularia-Arten, wie beispielsweise *Pyricularia oryzae*, Fusarium-Arten, wie beispielsweise *Fusarium culmorum,* Botrytis-Arten, Septoria-Arten, wie beispielsweise *Septoria nodorum,* Leptosphaeria-Arten, wie beispielsweise *Leptosphaeria nodorum,* Cercospora-Arten, wie beispielsweise *Cercospora canescens,* Alternaria-Arten, wie beispielsweise *Alternaria brassicae* oder Pseudocercosporella-Arten, wie beispielsweise *Pseudocercosporella herpotrichoides.*

Von besonderem Interesse sind z.B. auch *Magnaporthe grisea, Cochliobulus heterostrophus, Nectria hematococcus* and Phytophtora Arten.

Bevorzugt umfassen die erfindungsgemäßen Polypeptide damit eine Aminosäuresequenz aus pflanzenpathogenen Pilzen mit einer der Sequenz gemäß SEQ ID NO: 2 oder SEQ ID NO: 4

Der Ausdruck "Polypeptide", wie er hierin verwendet wird, bezieht sich sowohl auf kurze Aminosäureketten, die gewöhnlich als Peptide, Oligopeptide oder Oligomere bezeichnet werden, als auch auf längere Aminosäureketten, die gewöhnlich als Proteine bezeichnet werden. Er umfasst Aminosäureketten, die entweder durch natürliche Prozesse, wie posttranslationale Prozessierung, oder durch chemische Verfahren, die Stand der Technik sind, modifiziert sein können. Solche Modifikationen können an verschiedenen Stellen und mehrfach in einem Polypeptid vorkommen, wie beispielsweise am Peptid-Rückgrat, an der Aminosäure-Seitenkette, am Amino- und/oder am Carboxy-Terminus. Sie umfassen beispielsweise Acetylierungen, Acylierungen, ADP-Ribosylierungen, Amidierungen, kovalente Verknüpfungen mit Flavinen, Häm-Anteilen, Nukleotiden oder Nukleotid-Derivaten, Lipiden oder Lipid-Derivaten oder Phophatidylinositol, Cyclisierungen, Disulfidbrückenbildungen, Demethylierungen, Cystin-Bildungen, Formylierungen, gamma-Carboxylierungen, Glycosylierungen, Hydroxylierungen, Iodierungen, Methylierungen, Myristoylierungen, Oxidationen, proteolytische Prozessierungen, Phosphorylierungen, Selenoylierungen und tRNA-vermittelte Additionen von Aminosäuren.

Die Polypeptide können in der Form "reifer" Proteine oder als Teile größerer Proteine, z.B. als Fusionsproteine, vorliegen. Weiterhin können sie Sezernierungs- oder "Leader"-Sequenzen, Pro-Sequenzen, Sequenzen, die eine einfache Reinigung ermöglichen, wie mehrfache Histidin-Reste, oder zusätzliche stabilisierende Aminosäuren aufweisen. Die erfindungsgemäßen Proteine können ebenfalls so vorliegen, wie sie natürlicherweise in ihrem Herkunftsorganismus vorliegen, aus dem sie zum Beispiel direkt gewonnen werden können.

Die Polypeptide können im Vergleich zu den entsprechenden Regionen von natürlich vorkommenden IMPDHs Deletionen oder Aminosäuresubstitutionen aufweisen, solange sie zumindest noch die biologische Aktivität einer vollständigen IMPDH zeigen. Konservative Substitutionen sind bevorzugt. Solche konservativen Substitutionen umfassen Variationen, wobei eine Aminosäure durch eine andere Aminosäure aus der folgenden Gruppe ersetzt wird:

1. Kleine aliphatische, nicht-polare oder wenig polare Reste: Ala, Ser, Thr, Pro und Gly;
2. Polare, negativ geladene Reste und deren Amide: Asp, Asn, Glu und Gln;
3. Polare, positiv geladene Reste: His, Arg und Lys;
4. Große aliphatische, nicht-polare Reste: Met, Leu, Ile, Val und Cys; und
5. Aromatische Reste: Phe, Tyr und Trp.

Die folgende Liste zeigt bevorzugte konservative Substitutionen:

| **Ursprünglicher Rest** | **Substitution** |
|---|---|
| Ala | Gly, Ser |
| Arg | Lys |
| Asn | Gln, His |
| Asp | Glu |
| Cys | Ser |
| Gln | Asn |
| Glu | Asp |
| Gly | Ala, Pro |
| His | Asn, Gln |
| Ile | Leu, Val |
| Leu | Ile, Val |
| Lys | Arg, Gln, Glu |
| Met | Leu, Tyr, Ile |
| Phe | Met, Leu, Tyr |
| Ser | Thr |
| Thr | Ser |

(fortgesetzt)

| Ursprünglicher Rest | Substitution |
|---|---|
| Trp | Tyr |
| Tyr | Trp, Phe |
| Val | Ile, Leu |

Ein mögliches Reinigungsverfahren der IMPDH basiert auf präparativer Elektrophorese, FPLC, HPLC (z.B. unter Anwendung von Gelfiltrations-, Reversphasen- oder leicht hydrophoben Säulen), Gelfiltration, differentieller Präzipitation, Ionenaustausch-Chromatographie oder Affinitätschromatographie.

Ein schnelles Verfahren zum Isolieren der erfindungsgemäßen Polypeptide, die von Wirtszellen unter Verwendung einer erfindungsgemäß zu verwendenden Nukleinsäure synthetisiert werden, beginnt mit der Expression eines Fusionsproteins, wobei der Fusionspartner auf einfache Weise affinitätsgereinigt werden kann. Der Eusionspartner kann beispielsweise ein 6xHis-Tag sein. Das Fusionsprotein kann dann an einer Nickel-NTA-Affinitätssäule gereinigt werden. Der Fusionspartner kann durch partielle proteolytische Spaltung beispielsweise an Linkern zwischen dem Fusionspartner und dem zu reinigenden erfindungsgemäßen Polypeptid abgetrennt werden. Der Linker kann so gestaltet werden, dass er Ziel-Aminosäuren, wie Arginin- und Lysin-Reste einschließt, die Stellen für eine Spaltung durch Trypsin definieren. Um solche Linker zu erzeugen, können Standard-Klonierungsverfahren unter Verwendung von Oligonukleotiden angewendet werden.

Weitere mögliche Reinigungsverfahren basieren wiederum auf präparativer Elektrophorese, FPLC, HPLC (z. B. unter Anwendung von Gelfiltrations-, Reversphasen- oder leicht hydrophoben Säulen), Gelfiltration, differentieller Präzipitation, Ionenaustausch-Chromatographie und Affinitätschromatographie.

Die Ausdrücke "Isolierung oder Reinigung", wie sie hierin verwendet werden, bedeuten, dass die erfindungsgemäßen Polypeptide von anderen Proteinen oder anderen Makromolekülen der Zelle oder des Gewebes abgetrennt werden. Vorzugsweise ist eine die erfindungsgemäßen Polypeptide enthaltende Zusammensetzung hinsichtlich des Proteingehalts gegenüber einer Präparation aus den Wirtszellen mindestens 10-fach und besonders bevorzugt mindestens 100-fach angereichert.

Die erfindungsgemäßen Polypeptide können auch ohne Fusionspartner mit Hilfe von Antikörpern, die an die Polypeptide binden, affinitätsgereinigt werden.

Gegenstand der vorliegenden Anmeldung ist damit ebenfalls ein Verfahren zum Herstellen des Polypeptids Imp1 mit der SEQ ID NO: 2 oder SEQ ID NO: 4 oder dazu homologer Polypeptide aus pflanzenpathogenen Pilzen mit der Aktivität einer IMPDH, welches gekennzeichnet ist durch

(a) das Kultivieren einer Wirtszelle enthaltend zumindest eine exprimierbare Nukleinsäuresequenz kodierend für ein Polypeptid aus pflanzenpathogenen Pilzen mit der biologischen Aktivität einer IMPDH unter Bedingungen, die die Expression dieser Nukleinsäure gewährleisten, oder

(b) das Exprimieren einer exprimierbaren Nukleinsäuresequenz kodierend für ein Polypeptid aus pflanzenpathogenen Pilzen mit der biologischen Aktivität einer IMPDH in einem *in vitro*-System, und

(c) die Gewinnung des Polypeptids aus der Zelle, dem Kulturmedium oder dem *in vitro*-System.

Insbesondere ist Gegenstand der vorliegenden Erfindung ein Verfahren zum Herstellen des Polypeptids Imp1 mit der SEQ ID NO: 2 oder SEQ ID NO: 4 oder dazu homologer Polypeptide mit der Aktivität einer IMPDH aus pflanzenpathogenen Pilzen, wobei

(a) eine exprimierbare Nukleinsäuresequenz kodierend für ein Polypeptid aus pflanzenpathogenen Pilzen mit der biologischen Aktivität einer IMPDH in geeignete Wirtszellen transformiert wird,

(b) die transformierten Zellen bei einer geeigneten Temperatur inkubiert werden, wobei dem Medium 0,2 % Glucose zugesetzt werden,

(c) die Zellen nach einem für ein ausreichendes Wachstum der Zellen geeigneten Zeitraum induziert werden,

(d) die Zellen nach einem für eine ausreichende Expression der unter (a) genannten Nukleinsäure geeigneten Zeitraum geerntet werden, und gegebenenfalls

(e) das Polypeptid aus den geernteten Zellen isoliert und gereinigt wird.

Die so erhaltenen Zellen enthaltend das erfindungsgemäße Polypeptid oder das so erhaltene gereinigte Polypeptid sind geeignet, in Verfahren zum Identifizieren von Modulatoren bzw. Inhibitoren der IMPDH verwendet zu werden.

Gegenstand der vorliegenden Erfindung ist somit auch die Verwendung von Polypeptiden aus pflanzenpathogenen Pilzen, welche zumindest eine biologische Aktivität einer IMPDH ausüben und eine Aminosäuresequenz ausgewählt aus den Sequenzen, gemäß SEQ ID NO: 2 oder SEQ ID NO: 4 in Verfahren zum Identifizieren von Inhibitoren eines Polypeptids aus pflanzenpathogenen Pilzen mit der Aktivität einer IMPDH, wobei die Inhibitoren der IMPDH als Fungizide verwendet werden können.

Besonders bevorzugt ist die Verwendung von Polypeptiden aus pflanzenpathogenen Basidiomyceten, besonders aus der Gattung *Ustilago*, insbesondere aus *Ustilago maydis*, wobei hier das Polypeptid gemäß SEQ ID NO: 2 oder SEQ ID NO: 4 besonders bevorzugt ist, in Verfahren zum Identifizieren von Inhibitoren eines Polypeptids aus pflanzenpathogenen Pilzen mit der Aktivität einer IMPDH, wobei die Inhibitoren der IMPDH als Fungizide verwendet werden können.

Fungizide Wirkstoffe, die mit Hilfe einer der erfindungsgemäßen IMPDHs gefunden werden, können also auch mit IMPDHs aus anderen phytopathogenen Pilzspezies interagieren, wobei die Interaktion mit den unterschiedlichen in diesen Pilzen vorkommenden IMPDHs nicht immer gleich stark sein muss. Dies erklärt unter anderem die beobachtete Selektivität wirksamer Substanzen.

Wie bereits vorstehend erläutert, ermöglicht die Verwendung der erfindungsgemäßen Nukleinsäuren bzw. Polypeptide in einem geeigneten Verfahren das Auffinden von Verbindungen, die an die erfindungsgemäßen Polypeptide binden und/oder die Verbindung inhibieren. Diese können dann als Fungizide bei Pflanzen angewandt werden.

Gegenstand der vorliegenden Erfindung ist deshalb auch ein Verfahren, dass zur Identifizierung von fungiziden Wirkstoffen geeignet ist, die an die erfindungsgemäßen Polypeptide binden und/oder deren biologische Aktivität modulieren, d.h. aktivieren oder inhibieren.

Verfahren, die geeignet sind, Modulatoren, d.h. Aktivatoren oder Inhibitoren bzw. Agonisten oder Antagonisten der erfindungsgemäßen Polypeptide zu identifizieren, beruhen in aller Regel auf der Bestimmung der Aktivität bzw. der biologischen Funktionalität des Polypeptids. Dazu kommen prinzipiell sowohl auf ganzen Zellen beruhende Verfahren *(in vivo* Verfahren) in Frage, wie auch Verfahren, die auf der Verwendung des aus den Zellen isolierten Polypeptids beruhen, das in gereinigter oder teilweise gereinigter Form oder auch als Rohextrakt vorliegen kann. Diese zellfreien *in vitro* Verfahren können ebenso wie *in vivo* Verfahren im Labormaßstab, in bevorzugter Weise aber auch in HTS oder UHTS Verfahren genutzt werden.

Viele Testsysteme, die die Prüfung von Verbindungen und natürlichen Extrakten zum Ziel haben, sind bevorzugt auf hohe Durchsatzzahlen ausgerichtet, um die Zahl der untersuchten Substanzen in einem gegebenen Zeitraum zu maximieren. Testsysteme, die auf zellfreiem Arbeiten beruhen, brauchen gereinigtes oder semigereinigtes Protein. Sie sind geeignet für eine "erste" Prüfung, die in erster Linie darauf abzielt, einen möglichen Einfluss einer Substanz auf das Zielprotein zu detektieren. Ist eine solche erste Prüfung erfolgt und eine oder mehrere Verbindungen, Extrakte etc. gefunden, kann die Wirkung solcher Verbindungen im Labor noch gezielter untersucht werden. So kann in einem ersten Schritt die Inhibierung oder Aktivierung des erfindungsgemäßen Polypeptids *in vitro* noch einmal geprüft werden, um im Anschluss daran die Wirksamkeit der Verbindung am Zielorganismus, hier einem oder mehreren pflanzenpathogenen Pilzen, zu testen. Die Verbindung kann dann gegebenenfalls als Ausgangspunkt für die weitere Suche und Entwicklung von fungiziden Verbindungen verwendet werden, die auf der ursprünglichen Struktur basieren, jedoch z. B. hinsichtlich Wirksamkeit, Toxizität oder Selektivität optimiert sind.

Um Modulatoren aufzufinden, kann z.B. ein synthetischer Reaktionsmix (z.B. Produkte der *in vitro* Transkription) oder ein zellulärer Bestandteil, wie eine Membran, ein Kompartiment oder irgendeine andere Präparation, die die erfindungsgemäßen Polypeptide enthält, zusammen mit einem gegebenenfalls markierten Substrat oder Liganden der Polypeptide in Gegenwart und Abwesenheit eines Kandidatenmoleküls, das ein Agonist oder Antagonist sein kann, inkubiert werden. Die Fähigkeit des Kandidatenmoleküls die Aktivität der erfindungsgemäßen Polypeptide zu erhöhen oder zu hemmen, wird z.B. erkennbar an einer erhöhten oder verringerten Bindung des gegebenenfalls markierten Liganden oder an einer erhöhten oder verringerten Umsetzung des gegebenenfalls markierten Substrates. Moleküle, die zu einer erhöhten Aktivität der erfindungsgemäßen Polypeptide führen, sind Agonisten. Moleküle, die die biologische Aktivität der erfindungsgemäßen Polypeptide hemmen, sind gute Antagonisten.

Die Detektion der biologischen Aktivität der erfindungsgemäßen Polypeptide kann durch ein so genanntes Reportersystem verbessert werden. Reportersysteme in dieser Hinsicht umfassen, sind aber nicht beschränkt auf colorimetrisch oder fluorimetrische nachweisbare Substrate, die in ein Produkt umgewandelt werden oder ein Reportergen, das auf Veränderungen der Aktivität oder der Expression der erfindungsgemäßen Polypeptide anspricht oder andere bekannte Bindungstests.

Ein weiteres Beispiel für ein Verfahren, mit welchem Modulatoren der erfindungsgemäßen Polypeptide aufgefunden werden können, ist ein Verdrängungstest, bei dem man unter dafür geeigneten Bedingungen die erfindungsgemäßen Polypeptide und einen potenziellen Modulator mit einem Molekül, das bekanntermaßen an die erfindungsgemäßen Polypeptide bindet, wie einem natürlichen Substrat oder Liganden oder einem Substrat- oder Liganden-Mimetikum zusammenbringt. Die erfindungsgemäßen Polypeptide selbst können markiert werden, z.B. fluorimetrisch oder colorimetrisch, so dass man die Anzahl der Polypeptide, die an einen Liganden gebunden sind oder die eine Umsetzung mitgemacht haben, exakt bestimmen kann. Ebenso kann jedoch die Bindung mittels des gegebenenfalls markierten Substrats, Liganden bzw. Substratanalogen verfolgt werden. Auf diese Weise lässt sich die Effektivität eines Agonisten oder Antagonisten ermessen.

Effekte wie Zelltoxizität werden in diesen *in vitro* Systemen in der Regel ignoriert. Die Testsysteme überprüfen dabei sowohl inhibierende bzw. suppressive Effekte der Substanzen, als auch stimulatorische Effekte. Die Effektivität einer Substanz kann durch konzentrationsabhängige Testreihen überprüft werden. Kontrollansätze ohne Testsubstanzen bzw. ohne Enzym können zur Bewertung der Effekte herangezogen werden.

Durch die anhand der vorliegenden Erfindung verfügbaren, für eine erfindungsgemäße IMPDH kodierende Nukleinsäuren enthaltenden Wirtszellen, wird die Entwicklung von Testsystemen, die auf Zellen basieren, zur Identifizierung von Substanzen ermöglicht, die die Aktivität der erfindungsgemäßen Polypeptide modulieren.

Eine andere Möglichkeit zur Identifizierung von Substanzen, die die Aktivtät der erfindungsgemäßen Polypeptide modulieren, ist so auch der sogenannten "Scintillation Proximity Assay" (SPA), siehe EP 015 473. Dieses Testsystem nutzt die Interaktion eines Polypeptids (z.B. IMPDH aus *U. maydis*) mit einem radiomarkierten Liganden bzw. Substrat. Das Polypeptid ist dabei an kleine Kügelchen ("Microspheres") oder Perlen ("Beads") gebunden, die mit szintillierenden Molekülen versehen sind. Im Verlauf des Abfalls der Radioaktivität wird die szintillierende Substanz im Kügelchen durch die subatomaren Partikel des radioaktiven Markers angeregt und ein detektierbares Photon emittiert. Die Testbedingungen werden so optimiert, dass nur jene vom Liganden ausgehenden Partikel zu einem Signal führen, die von einem an das erfindungsgemäße Polypeptid gebundenen Liganden ausgehen.

Vorzugsweise handelt es sich bei den zu identifizierenden Modulatoren um kleine organisch-chemische Verbindungen.

Ein Verfahren zum Identifizieren einer Verbindung, die die Aktivität einer IMPDH aus pflanzenpathogenen Pilzen moduliert und die als Fungizid im Pflanzenschutz verwendet werden kann, besteht demnach darin, dass man

a) ein erfindungsgemäßes Polypeptid oder eine Wirtszelle enthaltend dieses Polypeptid mit einer chemischen Verbindung oder mit einem Gemisch von chemischen Verbindungen unter Bedingungen in Kontakt bringt, die die Interaktion einer chemischen Verbindung mit dem Polypeptid erlauben,

b) die Aktivität des erfindungsgemäßen Polypeptids bei Abwesenheit einer chemischen Verbindung mit der Aktivität des erfindungsgemäßen Polypeptids bei Anwesenheit einer chemischen Verbindung oder eines Gemisches von chemischen Verbindungen vergleicht, und

c) die chemische Verbindung bestimmt, die die Aktivität des erfindungsgemäßen Polypeptids spezifisch moduliert.

Besonders bevorzugt wird dabei diejenige Verbindung bestimmt, die die Aktivität des erfindungsgemäßen Polypeptids spezifisch inhibiert. Der Begriff "Aktivität", wie er hier verwendet wird, bezieht sich auf die biologische Aktivität des erfindungsgemäßen Polypeptids.

Die Aktivität bzw. die Ab- oder Zunahme der Aktivität des erfindungsgemäßen Polypeptids wird bevorzugt mittels der Umsetzung des Substrats $NAD^+$ zu NADH bestimmt. Dabei wird die geringere bzw. inhibierte Aktivität des erfindungsgemäßen Polypeptids anhand der photospektrometrischen Bestimmung der Abnahme des $NAD^+$ bzw. der Zunahme des NADH verfolgt. Aufgrund der Lichtabsorption des Nicotinamidringes besitzt das reduzierte Nicotinamid-Coenzym NADH nämlich ein Absorptionsmaximum bei 340 nm. Die oxidierte Form $NAD^+$ zeigt dagegen zwischen 300 und 400 nm keine Absorption. Die erfindungsgemäße enzymatische Reaktion, die zu einer Reduktion des $NAD^+$ führt, kann deshalb anhand der Zunahme der Absorption z.B. bei 340 nm verfolgt werden. Der Einfluss eines Inhibitors auf die Reaktion kann damit ebenfalls bestimmt werden.

Eine weitere Möglichkeit zur Bestimmung der Aktivität bzw. der Ab- oder Zunahme der Aktivität mittels der Umsetzung des Substrats $NAD^+$ zu NADH besteht im Nachweis des entstehenden NADHs in einem gekoppelten Luziferase-Assay. Dabei erfolgt eine Kopplung der Reaktion mit der NADH-FMN Oxidoreduktase nach folgendem Schema:

$$FMNH_2 + RCHO + O_2 \rightarrow FMN + RCOOH + H_2O + h\nu,$$

wobei R = Alkyl (Baldwin et al. (1975): Bacterial Luciferase. Binding of Oxidized Flavin Mononucleotide. *J. Biol. Chem.*,

250, 2763). Dabei ist das NADH der primäre Elektronendonor. Die Elektronen werden dann über das FMN auf die Luziferase weitergeleitet, wobei es zur Emission von Licht kommt. Das in der erfindungsgemäßen Reaktion entstehende NADH kann in einem Bereich von 1 bis 25μM gut nachgewiesen werden. Die Lichtausbeute der Luziferase ist deutlich pH-abhängig, wobei ein Bereich von pH 7 bis pH 8,5 besonders geeignet ist.

Die Aktivität bzw. die Ab- oder Zunahme der Aktivität kann auch bevorzugt mittels der Umsetzung des Substrats $NAD^+$ zu NADH bestimmt werden, wobei die geringere bzw. inhibierte Aktivität des erfindungsgemäßen Polypeptids anhand eines geringeren Anstiegs der Fluoreszenz bestimmt wird, die vom im geringeren Maße entstehenden NADH (siehe auch Abb. 1) ausgeht. NADH fluoresziert stärker als $NAD^+$.

Dazu wird das erfindungsgemäße Polypeptid in einer geeigneten Konzentration, die vorzugsweise zwischen 0,1 und 20 ng/μl, bevorzugt zwischen 0,13 und 10 ng/μl des Enzyms liegt, mit $NAD^+$ in einer Konzentration von 5 bis 400 μM, bevorzugt 10 bis 100 μM, und IMP in einer Konzentration von 5 bis 400 μM, bevorzugt von 50 bis 300μM, bei geeigneter Temperatur inkubiert. Die Konzentration des IMP ist bevorzugt jeweils doppelt so hoch wie die des Substrats $NAD^+$. Zu beachten ist, dass für beide Substrate, $NAD^+$ und IMP, eine Substratinhibition beobachtet werden kann, die verwendeten Konzentrationen der beiden Substrate also daraufhin geprüft werden sollten. Die Temperatur kann dabei in einem Bereich von 8 bis 37°C liegen. Die Messung erfolgt bevorzugt bei 10 bis 30°C, insbesondere ist auch Raumtemperatur geeignet. Die Reaktion erfolgt in einem gängigen Puffer ("Testpuffer"), der vorzugsweise z.B. 20 bis 200 mM KCl, 1 bis 5 mM DTE oder DTT, 0,01 bis 1 % BSA, 0,01 bis 0,1 % Tween 20 und 2 bis 10 % Glycerin enthalten und z.B. mit 25 bis 100mM Tris-Puffer auf einen pH-Wert von 8 eingestellt werden kann (siehe Beispiel 2). Es sind jedoch auch andere Pufferzusammensetzungen denkbar, in denen die erfindungsgemäßen Polypeptide ihre biologische Aktivität noch besitzen und die von ihnen katalysierte Reaktion ausführen können.

Die Bildung von NADH im Verlauf der Reaktion wird dann anhand der Fluoreszenz bei einer Anregungswellenlänge von 360 nm und einer Emissionswellenlänge von 465 nm verfolgt. Die Fluoreszenzerhöhung bei Anwesenheit einer chemischen Verbindung kann dann mit der Fluoreszenzerhöhung bei Abwesenheit einer chemischen Verbindung verglichen werden. Der Vergleich zeigt dann, ob bei Anwesenheit einer chemischen Verbindungen die Zunahme der Fluoreszenz geringer oder gegebenenfalls höher ist als bei Abwesenheit der chemischen Verbindung, d.h. ob die besagte Verbindung eine aktivatorische oder inhibitorische Wirkung auf das getestete Polypeptid hat. Der Zeitraum, in dem die Zunahme der Fluoreszenz gemessen wird, kann dabei variiert werden. Es kann ein langsamer Anstieg der Fluoreszenz im Verlauf mehrerer Stunden beobachtet werden, bis schließlich nach 5 bis 10 Stunden ein Plateau erreicht wird (siehe auch Abb. 3). Danach nimmt die Fluoreszenz in der Regel ab. Diese langsame Umsetzungsrate ist jedoch aus der Literatur bekannt und scheint für IMP Dehydrogenasen charakteristisch zu sein. Wichtig ist deshalb jedoch, den Puffer, in dem das Enzym gelagert bzw. die Reaktion durchgeführt wird so zu wählen, dass das erfindungsgemäße Polypeptid möglichst gut stabilisiert wird. Ein solcher Puffer sollte z.B. KCI, BSA, DTT oder DTE und Glycerin in einer geeigneten Konzentration enthalten, Der pH-Wert wird vorzugsweise auf pH 8 eingestellt. Bevorzugt werden 10-200 mM KCl, 10-200 mM Tris, 0,02-0,2 % BSA und 0,1-5 mM DTE oder DTTsowie 12-25% Glycerin verwendet.

Die Messung kann auch in für HTS- oder UHTS-Assays gängigen Formaten erfolgen, z.B. in Mikrotiterplatten, in denen z.B. ein Gesamtvolumen von 5 bis 50 μl pro Ansatz bzw. pro Well vorgelegt wird und die einzelnen Komponenten in einer der vorstehend angegebenen Endkonzentrationen vorliegen. Dabei wird die zu testende, potentiell die Aktivität des Enzyms inhibierende oder aktivierende Verbindung (Kandidatenmolekül) z.B. in einer geeigneten Konzentration im oben angegebenen Testpuffer enthaltend IMP vorgelegt. Dann wird das erfindungsgemäße Polypeptid in oben genanntem Testpuffer, enthaltend das zweite Substrat $NAD^+$, zugegeben und die Reaktion damit gestartet. Der Ansatz wird dann z.B. bis zu 2 oder 3 Stunden bei einer geeigneten Temperatur inkubiert und die Fluoreszenzzunahme bei einer Anregungswellenlänge von 360 nm und einer Emissionswellenlänge von 460 nm gemessen.

Eine weitere Messung erfolgt in einem entsprechenden Ansatz, jedoch ohne Zugabe eines Kandidatenmoleküls und ohne Zugabe eines erfindungsgemäßen Polypeptids (Negativkontrolle). Eine weitere Messung erfolgt wiederum bei Abwesenheit eines Kandidatenmoleküls, jedoch bei Anwesenheit des erfindungsgemäßen Polypeptids (Positivkontrolle). Negativ- und Positivkontrolle ergeben damit die Vergleichswerte zu den Ansätzen bei Anwesenheit eines Kandidatenmoleküls.

Um optimale Bedingungen für ein Verfahren zum Identifizieren von Inhibitoren der IMPDH bzw. zur Bestimmung der Aktivität der erfindungsgemäßen Polypeptide zu ermitteln, kann es vorteilhaft sein, den jeweiligen $K_M$-Wert des verwendeten erfindungsgemäßen Polypeptids zu bestimmen. Dieser gibt Aufschluss über die bevorzugt zu verwendende Konzentration des bzw. der Substrate. Im Fall der IMPDH aus *U. maydis* konnte ein $K_M$ von 25 μM bestimmt werden (siehe Abbildung 4).

Mit Hilfe des vorstehend beispielhaft beschriebenen Verfahrens konnte Verbindungen identifiziert werden, die die erfindungsgemäßen IMP Dehydrogenasen, insbesondere die IMPDH aus *U. maydis* gemäß SEQ ID NO: 2 oder SEQ ID NO: 4 inhibieren (siehe Beispiel 1 und 3).

In Tabelle I werden beispielhaft Verbindungen gezeigt, die mit dem erfindungsgemäßen Verfahren als Inhibitoren der IMPDH identifiziert werden konnten.

Der dort angegebene pI50-Wert ist der negative dekadische Logarithmus des so genannten IC50-Werts, der die molare Konzentration einer Substanz angibt, die zur 50 %igen Hemmung des Enzyms führt.

Ein pI50-Wert von 8 entspricht z.B. einer halbmaximalen Hemmung des Enzyms bei einer Konzentration von 10 nM.

**Tabelle II**

| Beispiel | Verbindung | pI50 |
|---|---|---|
| 1 | 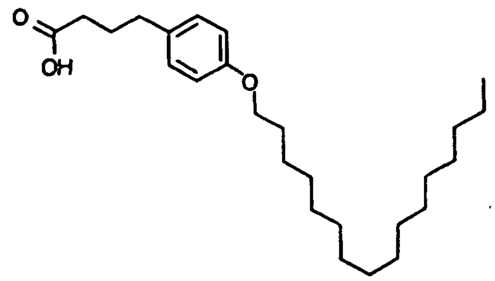 | 4,6 |
| 2 | 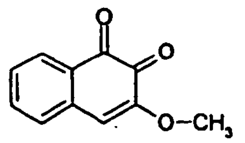 | 4,9 |
| 3 | 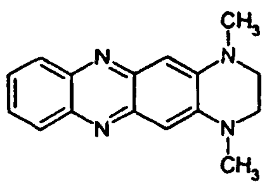 | 5,0 |
| 4 | 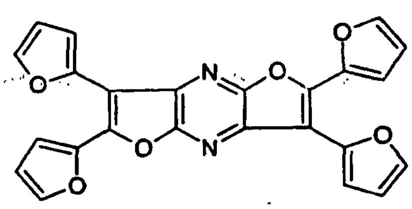 | 6,7 |

Im Rahmen der vorliegenden Erfindung konnte weiter gezeigt werden, dass die mit Hilfe eines erfindungsgemäßen Verfahrens identifizierten Inhibitoren einer erfindungsgemäßen IMPDH geeignet sind, pflanzenpathogene Pilze zu schädigen oder zu töten.

Dazu wurde z.B. in die Kavitäten von Mikrotiterplatten eine Lösung des zu prüfenden Wirkstoffs pipettiert. Nachdem das Lösungsmittel abgedampft war, wurde zu jeder Kavität Medium hinzugefügt. Das Medium wurde vorher mit einer geeigneten Konzentration von Sporen bzw. Mycel des zu prüfenden Pilzes versetzt.

Die resultierenden Konzentrationen des Wirkstoffes betragen z.B. 0,1, 1, 10 und 100 ppm.

Die Platten wurden anschließend auf einem Schüttler bei einer Temperatur von 22°C inkubiert, bis in der unbehandelten Kontrolle ein ausreichendes Wachstum feststellbar war.

Die Auswertung erfolgte photometrisch bei einer Wellenlänge von 620 nm. Aus den Messdaten der verschiedenen Konzentrationen wurde die Wirkstoffdosis, die zu einer 50 %igen Hemmung des Pilzwachstums gegenüber der unbehandelten Kontrolle führt ($ED_{50}$) berechnet.

Gegenstand der vorliegenden Erfindung sind deshalb auch Modulatoren der erfindungsgemäßen Polypeptide, insbesondere der zur Modulation der IMPDH aus *U. maydis* gemäß SEQ ID NO: 2 oder SEQ ID NO: 4 geeigneten Verbindungen oder Extrakte, die mit Hilfe eines der in der vorliegenden Anmeldung beschriebenen Verfahren zum Identifizieren von Modulatoren der IMPDH gefunden werden.

Weiterhin umfasst die vorliegende Erfindung Verfahren zum Auffinden von chemischen Verbindungen, welche die Expression der erfindungsgemäßen Polypeptide verändern. Auch solche "Expressionsmodulatoren" können neue fungizide Wirkstoffe darstellen. Expressionsmodulatoren können kleine organisch-chemische Moleküle, Peptide oder Antikörper sein, die an die regulatorischen Regionen der für die erfindungsgemäßen Polypeptide kodierenden Nukleinsäuren binden. Weiterhin können Expressionsmodulatoren kleine organisch-chemische Moleküle, Peptide oder Antikörper sein, die an ein Molekül binden, welches wiederum an regulatorische Regionen der für die erfindungsgemäßen Polypeptide kodierenden Nukleinsäuren bindet, und dadurch deren Expression beeinflusst. Expressionsmodulatoren können auch Antisense-Moleküle sein.

Die vorliegende Erfindung bezieht sich ebenfalls auf die Verwendung von Modulatoren der erfindungsgemäßen Polypeptide bzw. deren Expression als Fungizide (s. Beispiel 3).

Gegenstand der vorliegenden Erfindung sind ebenfalls Expressionsmodulatoren der Inosin-Monophosphat Dehydrogenasen die mit Hilfe der vorstehend beschriebenen Verfahren zum Auffinden von Expressionsmodulatoren gefunden werden.

Gegenstand der Erfindung sind weiterhin Antikörper, die spezifisch an die erfindungsgemäßen Polypeptide oder Fragmente davon binden. Die Herstellung solcher Antikörper erfolgt auf die übliche Weise. Beispielsweise können solche Antikörper produziert werden durch die Injektion eines substantiell immunkompetenten Wirts mit einer für die Antikörperproduktion effektiven Menge eines erfindungsgemäßen Polypeptids oder eines Fragments davon und durch nachfolgende Gewinnung dieses Antikörpers. Weiterhin lässt sich in an sich bekannter Weise eine immortalisierte Zelllinie erhalten, die monoklonale Antikörper produziert. Die Antikörper können gegebenenfalls mit einem Nachweisreagenz markiert sein. Bevorzugte Beispiele für ein solches Nachweisreagenz sind Enzyme, radioaktiv markierte Elemente, fluoreszierende Chemikalien oder Biotin. Anstelle des vollständigen Antikörpers können auch Fragmente eingesetzt werden, die die gewünschten spezifischen Bindungseigenschaften zeigen.

## Beispiele

### Beispiel 1

Klonierung, Expression und Reinigung von *impl* bzw. IMP1 aus *Ustilago maydis*

Für die Klonierung von *impl* bzw. dessen Expression wurde der ORF aus mRNA aus *U. maydis* über genspezifische Primer gemäß SEQ ID NO: 5 und 6 amplifiziert. Die entsprechende cDNA, ein Amplikon von 1675 bp Länge, wurde in den Vektor pCRTOP2.1 von Invitrogen zwischenkloniert und anschließend über die durch die Primer eingeführten Schnittstellen *Eco*RV und *Not*I in den mit *Eco*RV und *Not*I geschnittenen Vektor pET32a (Novagen) kloniert. Das resultierende Plasmid p830 enthält die vollständige kodierende Sequenz von *impl* in einer N-terminalen Fusion mit dem Thioredoxin-Tag, das Bestandteil des Vektors ist. Das Impl Fusionsprotein besitzt eine berechnete Masse von 78 kD.

Für die heterologe Expression wurde das Plasmid p830 so in BL21::DE3 transformiert, dass der Transformationsansatz direkt als Vorkultur in 50 ml Selektionsmedium diente. Diese Zellen wurden über Nacht bei 37°C inkubiert und anschließend 1:100 in Selektionsmedium (LB-Medium mit 100 µg/ml Ampicillin) verdünnt. Um die basale Aktivität des T7 Promotors zu reprimieren, wurde dem Medium 0,2 % Glucose zugesetzt. Bei einer $OD_{600nm}$ von 0,8 - 1,0 wurde die Temperatur der Zellen auf 16°C gesenkt und nach 10 min. mit 1mM IPTG (Endkonzentration) induziert. Die Zellen wurden nach 1,5 h geerntet. Die Zellpellets konnten ohne Aktivitätsverlust mehrere Monate bei -80°C gelagert werden. Der Aufschluss erfolgte durch Sonifizieren in Lysepuffer (200 mM KCl, 10 mM Imidazol, 50 mM Tris-HCl, pH 8, 15 % Glycerin). Die Reinigung erfolgte nach dem Standartprotokoll des Herstellers für Ni-NTA Säulen (siehe auch Abb. 2). Anschließend wurde das gereinigte Protein in Lagerungspuffer (200 mM KCl, 50 mM Tris-HCl, pH 8, 0,1 % BSA, 1mM DTE, 17 % Glycerin) umgepuffert. Aus einem Liter Kulturmedium konnte so etwa 4 mg lösliches Protein isoliert werden, das in Verfahren zum Identifizieren von Modulatoren der IMPDH verwendet werden konnte.

### Beispiel 2

(A) Identifzierung von Modulatoren der IMP Dehydrogenase in 384-Well-MTP

Zur Identifizierung von Modulatoren der IMPDH aus *U. mydis* (IMP1) wurden 384-Well-Mikrotiterplatten von Greiner verwendet.

In die erste Spalte wurde die Negativ-Kontrolle pipettiert. Diese setzte sich zusammen aus 5 µl Testpuffer (100 mM KCl, 50 mM Tris/HCl, pH 8, 2mM DTE, 0,1 % BSA, 0,05 % Tween 20, 5 % Glycerin) mit 5 % DMSO, 20 µl Testpuffer mit 200 µM IMP und 25 µl Testpuffer mit 100 µM $NAD^+$.

In die zweite Spalte wurde die Positiv-Kontrolle pipettiert. Diese setzte sich zusammen aus 5µl Testpuffer mit 5 % DMSO, 20 µl Testpuffer mit 200 µM IMP sowie 2 ng/µl IMPDH und 25 µl Testpuffer mit 100 µM $NAD^+$.

In die verbleibenden Spalten wurde eine Prüfsubstanz in einer Konzentration von 2 µM in DMSO vorgelegt, wobei zum Verdünnen der Substanz auf ein Volumen von 5 µl der Testpuffer enthaltend 200 µM IMP verwendet wurde. Nach Zugabe von 20 µl Testpuffer enthaltend 200 µM IMP und 2 ng/µl IMPDH wurden zum Start der Reaktion 25 µl Testpuffer enthaltend 100 mM $NAD^+$ zugegeben. Es folgte Inkubation bei Raumtemperatur für 2 Stunden.

Die Messung des während der Reaktion entstehenden NADH erfolgte durch Bestimmung der absoluten Fluoreszenz in einem für MTP geeigneten Tecan Ultra Fluoreszenzspektrometer.

(B) Identifzierung von Modulatoren der IMP Dehydrogenase in 1536-Well-MTP

Das Verfahren wurde wie vorstehend beschrieben durchgeführt, wobei das Gesamtvolumen der Ansätze nun nur 8 µl betrug. Die einzelnen Komponenten wurden entsprechend angepasst.

**Beispiel 3**

Nachweis der fungiziden Wirkung der identifizierten Inhibitoren der IMPDH

In die Kavitäten von Mikrotiterplatten wurde eine methanolische Lösung des anhand eines erfindungsgemäßen Verfahrens identifizierten Wirkstoffs, versetzt mit einem Emulgator, pipettiert. Nachdem das Lösungsmittel abgedampft war, wurden je Kavität 200 µl Potatoe-Dextrose-Medium hinzugefügt. Das Medium wurde vorher mit geeigneten Konzentrationen von Sporen bzw. Mycelen des zu prüfenden Pilzes (siehe Tabelle III) versetzt.

Die resultierenden Konzentrationen des Wirkstoffs betrugen 0,1, 1, 10 und 100 ppm. Die resultierende Konzentration des Emulgators betrug 300 ppm.

Die Platten wurden anschließend auf einem Schüttler bei einer Temperatur von 22°C inkubiert, bis in der unbehandelten Kontrolle ein ausreichendes Wachstum feststellbar war. Die Auswertung erfolgte photometrisch bei einer Wellenlänge von 620 nm. Aus den Messdaten der verschiedenen Konzentrationen wird die Wirkstoffdosis, die zu einer 50%igen Hemmung des Pilzwachstums gegenüber der unbehandelten Kontrolle führt ($ED_{50}$), berechnet. Verbindung 3 aus Tabelle II zeigte bereits bei den in Tabelle III angegebenen Aufwandmengen eine entsprechende Wirkung.

Tabelle III

| Organismus | $ED_{50}$ [ppm] |
|---|---|
| *Bortytis cinerea* | 27,7 |
| *Giberella zeae* | 60,3 |
| *Phytophtora cryptogea* | 55,1 |
| *Septoria tritici* | 43,4 |

**Patentansprüche**

1. Verfahren zum Identifizieren von Fungiziden,
**dadurch gekennzeichnet, dass** man

(a) eine IMP Dehydrogenase aus pflanzenpathogenen Pilzen mit einer chemischen Verbindung oder einem Gemisch von chemischen Verbindungen in Kontakt bringt,

(b) die biologische Aktivität der IMP Dehydrogenase bei Anwesenheit der chemischen Verbindung oder des Gemisches chemischer Verbindungen mit der biologischen Aktivität der IMP Dehydrogenase bei Abwesenheit der chemischen Verbindung oder des Gemisches von Verbindungen vergleicht, und

(c) die chemische Verbindung bestimmt, welche die biologische Aktivität der IMP Dehydrogenase spezifisch inhibiert.

2. Verfahren gemäß Anspruch 1, **dadurch gekennzeichnet, dass** man die fungizide Wirkung der in Schritt (c) bestimmten Verbindung überprüft, indem man sie mit einem oder mehreren Pilzen in Kontakt bringt.

3. Verwendung einer IMP-Dehydrogenase aus pflanzenpathogenen Pilzen zum Auffinden von fungiziden Verbindungen.

4. Verfahren zum Bekämpfen von pflanzenpathogenen Pilzen, **dadurch gekennzeichnet, dass** man

(a) eine IMP Dehydrogenase aus pflanzenpathogenen Pilzen mit einer chemischen Verbindung oder einem Gemisch von chemischen Verbindungen in Kontakt bringt,

(b) die biologische Aktivität der IMP Dehydrogenase bei Anwesenheit der chemischen Verbindung oder des Gemisches chemischer Verbindungen mit der biologischen Aktivität der IMP Dehydrogenase bei Abwesenheit der chemischen Verbindung oder des Gemisches von Verbindungen vergleicht,

(c) die chemische Verbindung bestimmt, welche die biologische Aktivität der IMP Dehydrogenase spezifisch inhibiert, und

(d) die chemische Verbindung mit dem pflanzenpathogenen Pilz in Kontakt bringt.

5. Verfahren zum Bekämpfen von pflanzenpathogenen Pilzen, **dadurch gekennzeichnet, dass** man die Pilze mit einem Inhibitor einer pilzlichen IMPDH behandelt.

6. Verwendung eines Inhibitors einer IMP Dehydrogenase aus pflanzenpathogenen Pilzen zur Bekämpfung von pflanzenpathogenen Pilzen.

7. Nukleinsäure, kodierend für eine IMP Dehydrogenase aus pflanzenpathogenen Pilzen, umfassend eine Sequenz ausgewählt aus

a) der Sequenz gemäß SEQ ID NO: 1 oder SEQ ID NO: 3, und

b) Sequenzen, die für ein Polypeptid kodieren, welches die Aminosäuresequenz gemäß SEQ ID NO: 2 oder SEQ ID NO: 4 umfasst.

8. DNA-Konstrukt umfassend eine Nukleinsäure gemäß Anspruch 7 und einen heterologen Promotor.

9. Vektor umfassend eine Nukleinsäure gemäß Anspruch 7, oder ein DNA-Konstrukt gemäß Anspruch 8.

10. Vektor gemäß Anspruch 9, **dadurch gekennzeichnet, dass** die Nukleinsäure funktionell mit regulatorischen Sequenzen verknüpft ist, die die Expression der Nukleinsäure in pro- oder eukaryotischen Zellen gewährleisten.

11. Wirtszelle enthaltend eine Nukleinsäure gemäß Anspruch 7, ein DNA-Konstrukt gemäß Anspruch 8 oder einen Vektor gemäß Anspruch 9 oder 10.

**12.** Wirtszelle gemäß Anspruch 11, **dadurch gekennzeichnet, dass** es sich um eine prokaryotische Zelle handelt.

**13.** Wirtszelle gemäß Anspruch 11, **dadurch gekennzeichnet, dass** es sich um eine eukaryotische Zelle handelt.

**14.** Polypeptid aus pflanzenpathogenen Pilzen mit der biologischen Aktivität einer IMP Dehydrogenase, welches von einer Nukleinsäure gemäß Anspruch 7 kodiert wird.

**15.** Polypeptid aus pflanzenpathogenen Pilzen mit einer Aminosäuresequenz gemäß SEQ ID NO: 2 oder SEQ ID NO: 4.

**16.** Verfahren zum Herstellen eines Polypeptids gemäß Anspruch 14 oder 15, umfassend

(a) das Kultivieren einer Wirtszelle gemäß einem der Ansprüche 11 bis 13 unter Bedingungen, die die Expression der Nukleinsäure gemäß Anspruch 7 gewährleisten, oder

(b) das Exprimieren einer Nukleinsäure gemäß Anspruch 7 in einem *in vitro*-System, und

(c) die Gewinnung des Polypeptids aus der Zelle, dem Kulturmedium oder dem *in vitro*-System.

**17.** Antikörper, welcher spezifisch an ein Polypeptid gemäß Anspruch 14 oder 15 bindet.

**18.** Verfahren gemäß Anspruch 1 oder 2, **dadurch gekennzeichnet, dass** man Polypeptide gemäß Anspruch 14 oder 15 oder Wirtszellen gemäß einem der Ansprüche 11 bis 13 verwendet.

**19.** Verfahren zum Auffinden von Fungiziden, welche die Expression von Polypeptiden aus pflanzenpathogenen Pilzen mit der biologischen Aktivität einer IMP Dehydrogenase verändern, umfassend die folgenden Schritte:

(a) Inkontaktbringen einer Wirtszelle enthaltend eine Nukleinsäure kodierend für eine IMP Dehydrogenase aus pflanzenpathogenen Pilzen mit einer chemischen Verbindung oder einem Gemisch von chemischen Verbindungen,

(b) Bestimmen der Polypeptidkonzentration, und

(c) Bestimmen der Verbindung, welche die Expression des Polypeptids spezifisch beeinflusst.

## Claims

**1.** Method of identifying fungicides
**characterized in that**

(a) an IMP dehydrogenase from phytopathogenic fungi is brought into contact with a chemical compound or a mixture of chemical compounds,

(b) the biological activity of the IMP dehydrogenase in the presence of the chemical compound or the mixture of chemical compounds is compared with the biological activity of the IMP dehydrogenase in the absence of the chemical compound or the mixture of chemical compounds, and

(c) the chemical compound which specifically inhibits the biological activity of the IMP dehydrogenase is determined.

**2.** Method according to Claim 1, **characterized in that** the fungicidal action of the compound determined in step (c) is verified by bringing it into contact with one or more fungi.

**3.** Use of an IMP dehydrogenase from phytopathogenic fungi for finding fungicidal compounds.

**4.** Method of controlling phytopathogenic fungi, **characterized in that**

(c) an IMP dehydrogenase from phytopathogenic fungi is brought into contact with a chemical compound or a mixture of chemical compounds,

(d) the biological activity of the IMP dehydrogenase in the presence of the chemical compound or the mixture of chemical compounds is compared with the biological activity of the IMP dehydrogenase in the absence of the chemical compound or the mixture of chemical compounds, and

(c) the chemical compound which specifically inhibits the biological activity of the IMP dehydrogenase is determined.

(d) the chemical compound is brought into contact with the phytopathogenic fungus.

**5.** Method of controlling phytopathogenic fungi, **characterized in that** the fungi are treated with an inhibitor of a fungal IMPDH.

**6.** Use of an inhibitor of an IMP dehydrogenase from phytopathogenic fungi for controlling phytopathogenic fungi.

**7.** Nucleic acid encoding an IMP dehydrogenase from phytopathogenic fungi, encompassing a sequence selected from

a) the sequence as shown in SEQ ID NO: 1 or SEQ ID NO: 3, and

b) sequences which encode a polypeptide encompassing the amino acid sequence as shown in SEQ ID NO: 2 or SEQ ID NO: 4.

**8.** DNA construct encompassing a nucleic acid according to Claim 7 and a heterologous promoter.

**9.** Vector encompassing a nucleic acid according to Claim 7, or a DNA construct according to Claim 8.

**10.** Vector according to Claim 9, **characterized in that** the nucleic acid is linked operably to regulatory sequences which ensure the expression of the nucleic acid in prokaryotic or eukaryotic cells.

**11.** Host cell containing a nucleic acid according to Claim 7, a DNA construct according to Claim 8 or a vector according to Claim 9 or 10.

**12.** Host cell according to Claim 11, **characterized in that** it takes the form of a prokaryotic cell.

**13.** Host cell according to Claim 11, **characterized in that** it takes the form of a eukaryotic cell.

**14.** Polypeptide from phytopathogenic fungi with the biological activity of an IMP dehydrogenase which is encoded by a nucleic acid according to Claim 7.

**15.** Polypeptide from phytopathogenic fungi with an amino acid sequence as shown in SEQ ID NO: 2 or SEQ ID NO: 4.

**16.** Method of generating a polypeptide according to Claim 14 or 15, which comprises

(a) culturing a host cell according to one of Claims 11 to 13 under conditions which ensure the expression of the nucleic acid according to Claim 7, or

(b) expressing a nucleic acid according to Claim 7 in an *in-vitro* system, and

(c) recovering the polypeptide from the cell, the culture medium or the *in-vitro* system.

**17.** Antibody which binds specifically to a polypeptide according to Claim 14 or 15.

**18.** Method according to Claim 1 or 2, **characterized in that** polypeptides according to Claim 14 or 15 or host cells according to one of Claims 11 to 13 are used.

**19.** Method of finding fungicides which modify the expression of polypeptides from phytopathogenic fungi with the biological activity of an IMP dehydrogenase, which comprises the following steps:

(a) bringing a host cell containing a nucleic acid encoding an IMP dehydrogenase from phytopathogenic fungi into contact with a chemical compound or a mixture of chemical compounds,

(b) determining the polypeptide concentration, and

(c) determining the compound which specifically influences the expression of the polypeptide.

**Revendications**

**1.** Méthode d'identification de fongicides,
**caractérisée en ce que**

(a) on met en contact une IMP déshydrogénase issue de champignons phytopathogènes avec un composé chimique ou avec un mélange de composés chimiques,
(b) on compare l'activité biologique de l'IMP déshydrogénase en présence du composé chimique ou du mélange de composés chimiques avec l'activité biologique de l'IMP déshydrogénase en l'absence du composé chimique ou du mélange de composés, et
(c) on détermine le composé chimique qui inhibe spécifiquement l'activité biologique de l'IMP déshydrogénase.

**2.** Méthode suivant la revendication 1, **caractérisée en ce qu'**on éprouve l'action fongicide du composé déterminé dans l'étape (c) en le mettant en contact avec un ou plusieurs champignons.

**3.** Utilisation d'une IMP déshydrogénase issue de champignons phytopathogènes pour la recherche de composés fongicides.

**4.** Procédé de lutte contre des champignons phytopathogènes, **caractérisé en ce que**

(a) on met en contact une IMP déshydrogénase issue de champignons phytopathogènes avec un composé chimique ou un mélange de composés chimiques,
(b) on compare l'activité biologique de l'IMP déshydrogénase en présence du composé chimique ou du mélange de composés chimiques avec l'activité biologique de l'IMP déshydrogénase en l'absence du composé chimique ou du mélange de composés,
(c) on détermine le composé chimique qui inhibe spécifiquement l'activité biologique de l'IMP déshydrogénase, et
(d) on met en contact le composé chimique avec le champignon phytopathogène.

**5.** Procédé pour combattre des champignons phytopathogènes, **caractérisé en ce qu'**on traite les champignons avec un inhibiteur d'une IMPDH de champignon.

**6.** Utilisation d'un inhibiteur d'une IMP déshydrogénase issue de champignons phytopathogènes pour combattre des champignons phytopathogènes.

**7.** Acide nucléique, codant une IMP déshydrogénase issue de champignons phytopathogènes, comprenant une séquence choisie entre

a) la séquence selon SEQ ID N:°1 ou SEQ ID N:°3, et
b) des séquences qui codent un polypeptide qui comprend la séquence d'aminoacides selon SEQ ID N:°2 ou SEQ ID N:°4.

**8.** Produit d'assemblage d'ADN comprenant un acide nucléique selon la revendication 7 et un promoteur hétérologue.

**9.** Vecteur comprenant un acide nucléique selon la revendication 7, ou un produit d'assemblage d'ADN selon la revendication 8.

**10.** Vecteur selon la revendication 9, **caractérisé en ce que** l'acide nucléique est en liaison fonctionnelle avec des séquences régulatrices qui assurent l'expression de l'acide nucléique dans des cellules procaryotiques ou eucaryotiques.

**11.** Cellule-hôte contenant un acide nucléique selon la revendication 7, un produit d'assemblage d'ADN selon la revendication 8 ou un vecteur selon la revendication 9 ou 10.

**12.** Cellule-hôte suivant la revendication 11, **caractérisée en ce qu'**il s'agit d'une cellule procaryotique.

**13.** Cellule-hôte suivant la revendication 11, **caractérisée en ce qu'**il s'agit d'une cellule eucaryotique.

**14.** Polypeptide issu de champignons phytopathogènes ayant l'activité biologique d'une IMP déshydrogénase, qui est codé par un acide nucléique selon la revendication 7.

**15.** Polypeptide issu de champignons phytopathogènes présentant une séquence d'aminoacides selon SEQ ID N: 2 ou SEQ ID N: 4.

**16.** Procédé de production d'un polypeptide selon la revendication 14 ou 15, comprenant :

(a) la culture d'une cellule-hôte selon l'une des revendications 11 à 13 dans des conditions qui assurent l'expression de l'acide nucléique selon la revendication 7, ou
(b) l'expression d'un acide nucléique selon la revendication 7 dans un système *in vitro,* et
(c) l'obtention du polypeptide à partir de la cellule, du milieu de culture ou du système *in vitro.*

**17.** Anticorps qui se lie spécifiquement à un polypeptide selon la revendication 14 ou 15.

**18.** Méthode suivant la revendication 1 ou 2, **caractérisée en ce qu'**on utilise des polypeptides selon la revendication 14 ou 15 ou des cellules-hôtes selon l'une des revendications 11 à 13.

**19.** Méthode de recherche de fongicides qui modifient l'expression de polypeptides issus de champignons phytopathogènes possédant l'activité biologique d'une IMP déshydrogénase, qui comprend les étapes suivantes :

(a) mise en contact d'une cellule-hôte contenant un acide nucléique codant une IMP déshydrogénase issue de champignons phytopathogènes avec un composé chimique ou un mélange de composés chimiques,
(b) détermination de la concentration en polypeptide, et
(c) détermination du composé qui influe spécifiquement sur l'expression du polypeptide.

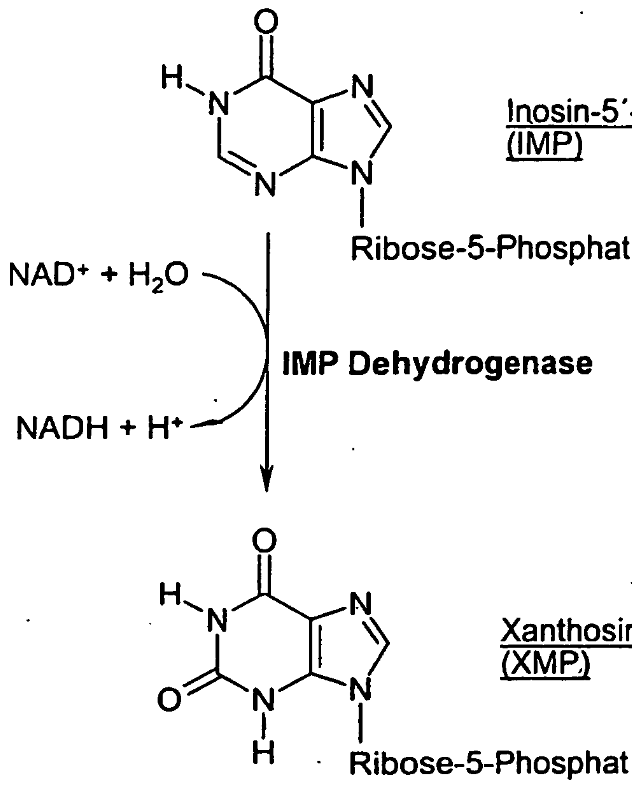


Inosin-5'-Monophosphat (IMP)

Xanthosin-5'-Monophosphat (XMP)

**Abbildung 1**

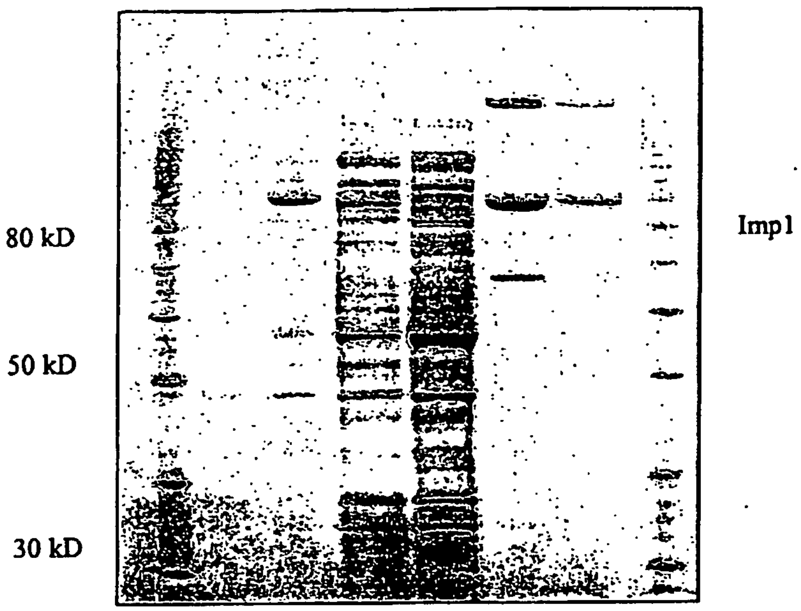


**Abbildung 2**

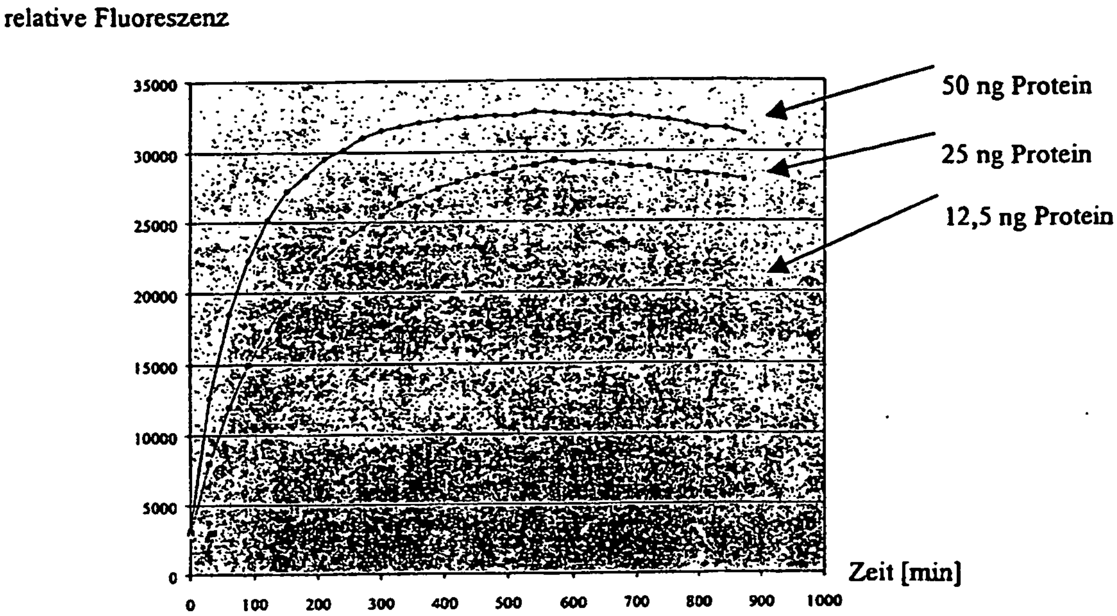
relative Fluoreszenz
35000
30000
25000
20000
15000
10000
5000
0
0
100
200
300
400
500
600
700
800
900
1000
Zeit [min]
50 ng Protein
25 ng Protein
12,5 ng Protein

**Abbildung 3**

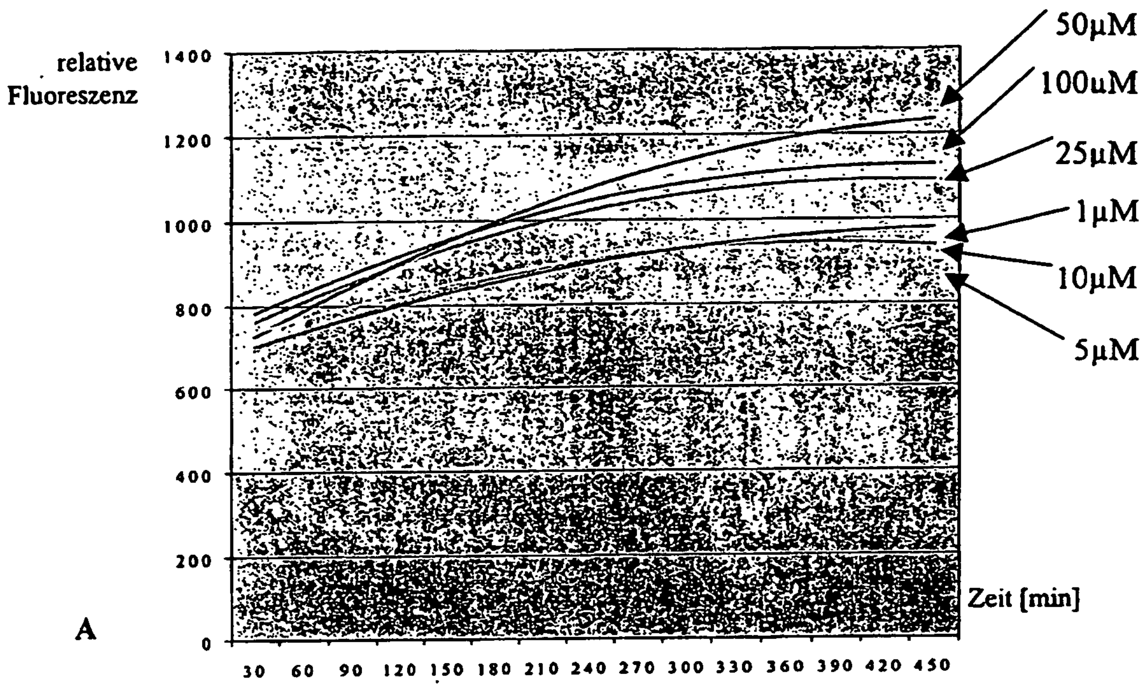


A

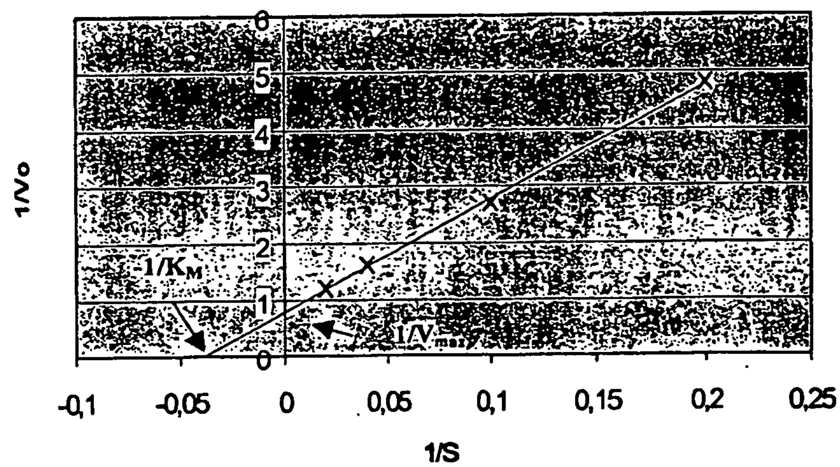


B

**Abbildung 4**

```
                 10         20         30         40         50
U.m.Impl   MPASNGIQLP QDEAVLSPSQ ALEHLKTYTY GDGLSMAELI DSRQHGGLTY
S.c.IMD3   ---------- MSAAPLDYKK ALEHLKTYSS KDGLSVQELM DSTTRGGLTY
S.c.IMD2   ---------- -MAAVRDYKT ALEFAKSLPR LDGLSVQELM DSKTRGGLTY
S.c.IMD1   ---------- -MAAIRDYKT ALDFTKSLPR PDGLSVQELM DSKIRGGLTY
H.s.IMD1   ---------- ----MADY-- LISGGTGYVP EDGLTAQQLF AS--ADDLTY
A.t.IMD    ---------- ---------- ------MSGF EDGFSAEKLF SQG--YSYTY
                 60         70         80         90        100
U.m.Impl   NDFLVLPGFI NFAASDVSLR TKVTKNVTLN TPFLSSPMDT VTETEMAIAM
S.c.IMD3   NDFLVLPGLV NFPSSAVSLQ TKLTKKITLN TPFVSSPMDT VTEADMAIYM
S.c.IMD2   NDFLVLPGLV DFPSSEVSLQ TKLTRNITLN TPFVSSPMDT VTESEMAIFM
S.c.IMD1   NDFLILPGLV DFASSEVSLQ TKLTRNITLN IPLVSSPMDT VTESEMATFM
H.s.IMD1   NDFLILPGFI DFIADEVDLT SALTRKITLK TPLISSPMDT VTEADMAIAM
A.t.IMD    DDVIFLPHFI DFSTDAVSLS TRLSKRVPLS IPCVASPMDT VSESHMAAAM
                110        120        130        140        150
U.m.Impl   GLMGGMGVIH NNMSPQEQAS VVRKVKKYEN GFITEPLCLD PKATVGDVLD
S.c.IMD3   ALLGGIGFIH HNCTPKEQAS MVKKVKMFEN GFINSPIVIS PTTTVGEVKV
S.c.IMD2   ALLGGIGFIH HNCTPEDQAD MVRRVKNYEN GFINNPIVIS PTTTVGEAKS
S.c.IMD1   ALLGGIGFIH HNCTPEDQAD MVRRVKNYEN GFINNPIVIS PTTTVGEAKS
H.s.IMD1   ALMGGIGFIH HNCTPEFQAN EVRKVKNFEQ GFITDPVVLS PSHTVGDVLE
A.t.IMD    AALGGIGIVH YNCDIDTQAS VIRHAKSLQV PIASDAVFKC PEHQIGSVDD
                160        170        180        190        200
U.m.Impl   VKERLGFGGI PITDTGAMHG KLLGIVTARD VQFR---DTT LPLSEVMTT-
S.c.IMD3   MKRKFGFSGF PVTEDGKCPG KLVGLVTSRD IQFLE--DDS LVVSEVMTK-
S.c.IMD2   MKERFGFSGF PVTEDGKRNG KLMGIVTSRD IQFVE--DNS LLVQDVMTK-
S.c.IMD1   MKEKYGFAGF PVTTDGKRNA KLVGVITSRD IQFVE--DNS LLVQDVMTK-
H.s.IMD1   AKMRHGFSGI PITETGTMGS KLVGIVTSRD IDFLAEKDHT TLLSEVMTPR
A.t.IMD    FGP---SSFV FVSQTGTLTP KLLGYVSKSE WSSMKDDQKE VKIYDYMKSC
                210        220        230        240        250
U.m.Impl   --DLVTAKQG VTLEQANTIL RDSKKGKLPI VDAEGRLVAL LARSDLLKNQ
S.c.IMD3   --NPVTGIKG ITLKEGNEIL KQTKKGKLLI VDDNGNLVSM LSRADLMKNQ
S.c.IMD2   --NPVTGAQG ITLSEGNEIL KKIKKGKLLI VDDNGNLVSM LSRTDLMKNQ
S.c.IMD1   --NPVTGAQG ITLSEGNEIL KKIKKGRLLV VDEKGNLVSM LSRTDLMKNQ
H.s.IMD1   -IELVVAPAG VTLKEANEIL QRSKKGKLPI VNDCDELVAI IARTDLKKNR
A.t.IMD    ENKDYYVPWD IDLDKIEAVL EDKQKG-FVV LEKEGETVNV VTKDDVERVK
                260        270        280        290        300
U.m.Impl   NFP-LASKRP ES-KQLYCAA AIGTRPSDRE RLSLLVEAGL DVVILDSSQG
S.c.IMD3   NYP-LASKSA TT-KQLLCGA AIGTIEADKE RLRLLVEAGL DVVILDSSQG
S.c.IMD2   NYP-LASKSA TT-KQLLCGA AIGTIDADKE RLRLLVEAGL DVVILDSSQG
S.c.IMD1   NYP-LASKSA NT-KQLLCGA SIGTMDADKE RLRLLVKAGL DVVILDSSQG
```

H.s.IMD1 DYP-LASKDS Q--KQLLCGA AVGTREDDKY RLDLLTQAGV DVIVLDSSQG

A.t.IMD GYPKLGSGTV GADKKWMVGA AIGTRESDKE RLEHLVKAGA NVVVLDSSQG

310 320 330 340 350

U.m.Imp1 NSVYQIEMIQ WIKQTYPQID VVAGNVVTRE QAASLIAAGA DALRVGMGSG

S.c.IMD3 NSVFQLNMIK WIKETFPDLE IIAGNVATRE QAANLIAAGA DGLRIGMGSG

S.c.IMD2 NSIFQLNMIK WIKETFPDLE IIAGNVATRE QAANLIAAGA DGLRIGMGSG

S.c.IMD1 NSIFELNMLK WVKESFPGLE VIAGNVVTRE QAANLIAAGA DGLRIGMGTG

H.s.IMD1 NSVYQIAMVH YIKQKYPHLQ VIGGNVVTAA QAKNLIDAGV DGLRVGMGCG

A.t.IMD NSIYQLEMIK YVKNTYPELD VVGGNVVTMY QAENLIKAGV DGLRVGMGSG

360 370 380 390 400

U.m.Imp1 SICITQEVMA VGRPQGTAVH AVAEFASKFG VPVIADGGIS NVGHIAKALA

S.c.IMD3 SICITQEVMA CGRPQGTAVY NVCQFANQFG VPCMADGGVQ NIGHITKALA

S.c.IMD2 SICITQEVMA CGRPQGTAVY NVCEFANQFG IPCMADGGVQ NIGHITKALA

S.c.IMD1 SICITQEVMA CGRPQGTAVY NVCEFANQFG VPCMADGGVQ NIGHITKALA

H.s.IMD1 SICITQEVMA CGRPQGTAVY KVAEYARRFG VPIIADGGIQ TVGHVVKALA

A.t.IMD SICTTQEVCA VGRGQATAVY KVSTLAAQHG VPVIADGGIS NSGHIVKALV

410 420 430 440 450

U.m.Imp1 LGASAVMMGG LLAGTNESPG DYFYRDGKRL KGYRGMGSIE AMEHQKKGKI

S.c.IMD3 LGSSTVMMGG MLAGTTESPG EYFYKDGKRL KAYRGMGSID AM--QKTG--

S.c.IMD2 LGSSTVMMGG MLAGTTESPG EYFYQDGKRL KAYRGMGSID AM--QKTG--

S.c.IMD1 LGSSTVMMGG MLAGTTESPG EYFYQDGKRL KAYRGMGSID AM--QKTG--

H.s.IMD1 LGASTVMMGS LLAATTEAPG EYFFSDGVRL KKYRGMGSLD AM--EKSS--

A.t.IMD LGASTVMMGS FLAGSTEAPG AYEYRNGRRV KKYRGMGSLE AM---TKG--

460 470 480 490 500

U.m.Imp1 AGATGKGAAK ADKVATDENA ATQRYFSESD AVKVAQGVAG AVQDKGSVKK

S.c.IMD3 ----NKG--- --------NA STSRYFSESD SVLVAQGVSG AVVDKGSIKK

S.c.IMD2 ----TKG--- --------NA STSRYFSESD SVLVAQGVSG AVVDKGSIKK

S.c.IMD1 ----TKG--- --------NA STSRYFSESD SVLVAQGVSG AVVDKGSIKK

H.s.IMD1 ---------- ---------S SQKRYFSEGD KVKIAQGVSG SIQDKGSIQK

A.t.IMD ---------- ---------- SDQRYLGDTA KLKIAQGVVG AVADKGSVLK

510 520 530 540 550

U.m.Imp1 FLPYLYTGLQ HSLQDMGVPH LYQLRSAVAS GQVRFELRTA SAQVEGGVHG

S.c.IMD3 FIPYLYNGLQ HSCQDIGCES LTSLKENVQN GEVRFEFRTA SAQLEGGVHN

S.c.IMD2 FIPYLYNGLQ HSCQDIGYKS LTLLKENVQS GKVRFEFRTA SAQLEGGVHN

S.c.IMD1 FIPYLYNGLQ HSCQDIGCRS LTLLKNNVQR GKVRFEFRTA SAQLEGGVHN

H.s.IMD1 FVPYLIAGIQ HGCQDIGARS LSVLRSMMYS GELKFEKRTM SAQIEGGVHG

A.t.IMD FIPYTMHAVK QGFQDLGASS LQSAHELLRD NTLRLEARTG AAQIEGGIHG

```
560 562
U.m.Imp1  LHSYEKRLFS SL
S.c.IMD3  LHSYEKRLYN --
S.c.IMD2  LHSYEKRLHN --
S.c.IMD1  LHSYEKRLHN --
H.s.IMD1  LHSYEKRLY- --
A.t.IMD   LVSYEKKSF- --
```

**Abbildung 5**